# EUROPEAN PATENT SPECIFICATION

(11) **EP 1 969 123 B1**
(45) Date of publication and mention of the grant of the patent: **12.07.2017**
(21) Application number: 06830937.6
(22) Date of filing: 15.12.2006
(51) Int. Cl.: C12N 9/42, C12N 15/62, C12N 15/56, D06M 16/00, C11D 3/386

(54) **NOVEL ENZYMES**
NEUE ENZYME
NOUVELLES ENZYMES

(30) Priority: 22.12.2005 FI 20055692; 22.12.2005 US 316397
(43) Date of publication of application: 17.09.2008
(73) Proprietor: AB Enzymes Oy, 05200 Rajamäki (FI)
(72) Inventor: VALTAKARI, Leena, FI-05200 Rajamäki (FI); ALAPURANEN, Marika, FI-05200 Rajamäki (FI); JÄMSÄ, Satu, FI-02910 Espoo (FI); SIIKA-AHO, Matti, FI-00810 Helsinki (FI); KALLIO, Jarno, FI-05200 Rajamäki (FI); VIIKARI, Liisa, FI-00200 Helsinki (FI); OJAPALO, Pentti, FI-04360 Tuusula (FI); VEHMAANPERÄ, Jari, FI-05200 Rajamäki (FI)
(74) Representative: Kolster Oy Ab
(86) International application number: PCT/FI2006/050560
(87) International publication number: WO 2007/071820

(56) References cited:
- EP-A- 1 291 431
- EP-A1- 1 291 431
- WO-A-00/20555
- WO-A-96/29397
- WO-A-98/12307
- WO-A-2004/053039
- WO-A1-96/29397
- WO-A1-98/12307
- WO-A2-00/20555
- WO-A2-2004/053039
- US-A1- 2002 168 751
- US-A1- 2002 168 751
- POOLE D M ET AL: "Characterization of hybrid proteins consisting of the catalytic domains of Clostridium and Ruminococcus endoglucanases, fused to Pseudomonas non-catalytic cellulose-binding domains" BIOCHEMICAL JOURNAL, THE BIOCHEMICAL SOCIETY, LONDON, vol. 279, 1 January 1991 (1991-01-01), pages 787-792, XP003014751 ISSN: 0264-6021
- TAKASHIMA S ET AL: "Comparison of gene structures and enzymatic properties between two endoglucanases from Humicola grisea" JOURNAL OF BIOTECHNOLOGY, ELSEVIER SCIENCE PUBLISHERS, AMSTERDAM, NL, vol. 67, no. 2-3, 22 January 1999 (1999-01-22), pages 85-97, XP004157885 ISSN: 0168-1656
- HOON KIM: "FUNCTIONAL ANALYSIS OF A HYBRID ENDOGLUCANASE OF BACTERIAL ORIGIN HAVING A CELLULOSE BINDING DOMAIN FROM A FUNGAL EXOGLUCANASE" APPLIED BIOCHEMISTRY AND BIOTECHNOLOGY, HUMANA PRESS, INC, US, vol. 75, no. 2-3, 1 December 1998 (1998-12-01), pages 193-204, XP008071913 ISSN: 0273-2289
- POOLE D.M. ET AL.: 'Characterization of hybrid proteins consisting of the catalytic domains of Clostridium and Ruminococcus endoglucanases, fused to Pseudomonas non-catalytic cellulose-binding domains' BIOCHEM. J. vol. 279, 1991, pages 787 - 792, XP003014751
- TAKASHIMA ET AL.: 'Comparison of gene structures and enzymatic properties between two endoglucanases from Humicola grisea' JOURNAL OF BIOTECHNOLOGY vol. 67, 1999, pages 85 - 97, XP004157885
- KIM ET AL.: 'Functional analysis of a hybrid endoglucanase of bacterial origin having a cellulose binding domain from a fungal exoglucanase' APPL. BIOCHEM. BIOTECHNOL. vol. 75, no. 2-3, 1998, pages 193 - 204, XP008071913

### SEQUENCE LISTING

<110> AB Enzymes Oy
<120> Novel enzymes
<130> 2052060PC
<160> 45
<170> PatentIn version 3.2
<210> 1
   <211> 2334
   <212> DNA
   <213> Acremonium thermophilum
<220>
   <221> misc_feature
   <222> (13) .. (13)
   <223> n means a, t, c, or g
<220>
   <221> CDS
   <222> (715)..(797)
<220>
   <221> Intron
   <222> (798)..(856)
<220>
   <221> CDS
   <222> (857)..(1105)
<220>
   <221> Intron
   <222> (1106)..(1228)
<220>
   <221> CDS
   <222> (1229)..(1787)
<400> 1
   tctgtctctt gtntcagaac agatctcctg gcggcctgct ttgccggtcc gaattgcgat     60
   cgatgcaacg tcgattgcat acgagctaag cccgtctcgt gataaccgca aggggtcttc    120
   cgagtttctg tctgcgaccc aggcattttc cgatttgtgt gcggggaccc aactgtcttc    180
   tggggagtac ctggtgacaa aagcacagat aaacagatgg atgacggtat tgctgtgata    240
   tcgccgtggc gctgaatcct ttctcttcgc taccaagata tttattcccc gttgtgaaat    300
   cttctattca gcccatccca tccggcaaca cgcatctgct tttcgttccg gcattccgat    360
   acctggttcc tggagtgcct accgagcctc gcttcctggg atcgggcgtt gcaccccgcc    420
   aaaccctatg ccccaaacgg tacggacaag gatgccggac cccggttttg tccagaaagg    480
   ttgcattcct acccacctcg ctggagccac aacatgcaga tcaccgcccg agggaggaca    540
   tgtgtggtgc agggacgttg gcaactctgc tgtgtctgaa gtatatgagg ccgatggttc    600
   tccttgcaca aagcagagaa tggagtagcc agctcctcct caccagagtc gcctttgcag    660
   cgtctcggca ttgcaggctc cccatcgtca gcatttcact tctcagcaac gaac atg      717
                                                               Met
                                                               1
   cgc tcc tca ccc ttt ctc cgc gca gct ctg gct gcc gct ctg cct ctg      765
   Arg Ser Ser Pro Phe Leu Arg Ala Ala Leu Ala Ala Ala Leu Pro Leu
               5                   10                  15
   agc gcc cat gcc ctc gac gga aag tcg acg ag  gtatgccaat cctcgtacct    817
   Ser Ala His Ala Leu Asp Gly Lys Ser Thr Arg
           20                  25
   ctgccctctg tagaaacaag tgaccgactg caaagacag a tac tgg gac tgc tgc     872
                                                Tyr Trp Asp Cys Cys
                                                    30
   aag ccg tcc tgc ggc tgg ccg gga aag gcc tcg gtg aac cag ccc gtc      920
   Lys Pro Ser Cys Gly Trp Pro Gly Lys Ala Ser Val Asn Gln Pro Val
       35                  40                  45
   ttc tcg tgc tcg gcc gac tgg cag cgc atc agc gac ttc aac gcg aag      968
   Phe Ser Cys Ser Ala Asp Trp Gln Arg Ile Ser Asp Phe Asn Ala Lys
   50                  55                  60                  65
   tcg ggc tgc gac gga ggc tcc gcc tac tcg tgc gcc gac cag acg ccc     1016
   Ser Gly Cys Asp Gly Gly Ser Ala Tyr Ser Cys Ala Asp Gln Thr Pro
                   70                  75                  80
   tgg gcg gtc aac gac aac ttc tcg tac ggc ttc gca gcc acg gcc atc     1064
   Trp Ala Val Asn Asp Asn Phe Ser Tyr Gly Phe Ala Ala Thr Ala Ile
               85                  90                  95
   gcc ggc ggc tcc gag tcc agc tgg tgc tgc gcc tgc tat gc              1105
   Ala Gly Gly Ser Glu Ser Ser Trp Cys Cys Ala Cys Tyr Ala
           100                 105                 110
   gtgagttctc tgcaagccgc ttcccacccc cgctttctgt gcaggccgct tcccccctac   1165
   ccacccactt cccccccccc gcctctgtga tcgggcatcc gagctaagtt gcgtgtcgtc   1225
   cag a ctc acc ttc aac tcg ggc ccc gtc gcg ggc aag acc atg gtg gtg   1274
         Leu Thr Phe Asn Ser Gly Pro Val Ala Gly Lys Thr Met Val Val
                   115                 120                 125
   cag tcg acc agc acc ggc ggc gac ctg ggc agc aac cag ttc gac ctc     1322
   Gln Ser Thr Ser Thr Gly Gly Asp Leu Gly Ser Asn Gln Phe Asp Leu
               130                 135                 140
   gcc atc ccc ggc ggc ggc gtg ggc atc ttc aac ggc tgc gcc tcc cag     1370
   Ala Ile Pro Gly Gly Gly Val Gly Ile Phe Asn Gly Cys Ala Ser Gln
           145                 150                 155
   ttc ggc ggc ctc ccc ggc gcc cag tac ggc ggc atc agc gac cgc agc     1418
   Phe Gly Gly Leu Pro Gly Ala Gln Tyr Gly Gly Ile Ser Asp Arg Ser
       160                 165                 170
   cag tgc tcg tcc ttc ccc gcg ccg ctc cag ccg ggc tgc cag tgg cgc      1466
   Gln Cys Ser Ser Phe Pro Ala Pro Leu Gln Pro Gly Cys Gln Trp Arg
   175                 180                 185                 190
   ttc gac tgg ttc cag aac gcc gac aac ccc acc ttc acc ttc cag cgc      1514
   Phe Asp Trp Phe Gln Asn Ala Asp Asn Pro Thr Phe Thr Phe Gln Arg
                   195                 200                 205
   gtg cag tgc ccg tcc gag ctc acg tcc cgc acg ggc tgt aag cgc gac      1562
   Val Gln Cys Pro Ser Glu Leu Thr Ser Arg Thr Gly Cys Lys Arg Asp
               210                 215                 220
   gac gac gcc agc tat ccc gtc ttc aac ccg cct agc ggt ggc tcc ccc      1610
   Asp Asp Ala Ser Tyr Pro Val Phe Asn Pro Pro Ser Gly Gly Ser Pro
           225                 230                 235
   agc acc acc agc acc acc acc agc tcc ccg tcc ggt ccc acg ggc aac      1658
   Ser Thr Thr Ser Thr Thr Thr Ser Ser Pro Ser Gly Pro Thr Gly Asn
       240                 245                 250
   cct cct gga ggc ggt ggc tgc act gcc cag aag tgg gcc cag tgc ggc      1706
   Pro Pro Gly Gly Gly Gly Cys Thr Ala Gln Lys Trp Ala Gln Cys Gly
   255                 260                 265                 270
   ggc act ggc ttc acg ggc tgc acc acc tgc gtc tcg ggc acc acc tgc      1754
   Gly Thr Gly Phe Thr Gly Cys Thr Thr Cys Val Ser Gly Thr Thr Cys
                   275                 280                 285
   cag gtg cag aac cag tgg tat tcc cag tgt ctg tgagcgggag ggttgttggg   1807
   Gln Val Gln Asn Gln Trp Tyr Ser Gln Cys Leu
               290                 295
   gtccgtttcc ctagggctga ggctgacgtg aactgggtcc tcttgtccgc cccatcacgg   1867
   gttcgtattc gcgcgcttag ggagaggagg atgcagtttg agggggccac attttgaggg   1927
   ggacgcagtc tggggtcgaa gcttgtcggt tagggctgcc gtgacgtggt agagcagatg   1987
   ggaccaagtg cggagctagg caggtgggtg gttgtggtgg tggcttacct tctgtaacgc   2047
   aatggcatct catctcactc gcctgctccc tgattggtgg ctctgttcgg cctggcgctt   2107
   tttgggaccg ctggctggaa tggattgctc cggaacgcca ggttgagctg ggctggcgcg   2167
   agtagattgg ccgctccgag ctgcaaccat aataaaattt tcggaccctg taagccgcac   2227
   ccgaccaggt ctccattggc ggacatgcac gacgtccttc gcaggcacgg cctgcccgcc   2287
   tctgatcacc cgcagttttc gtaccgtcag accagataca agccccg                 2334
<210> 2
   <211> 297
   <212> PRT
   <213> Acremonium thermophilum
<400> 2
   Met Arg Ser Ser Pro Phe Leu Arg Ala Ala Leu Ala Ala Ala Leu Pro
   1               5                   10                  15
   Leu Ser Ala His Ala Leu Asp Gly Lys Ser Thr Arg Tyr Trp Asp Cys
               20                  25                  30
   Cys Lys Pro Ser Cys Gly Trp Pro Gly Lys Ala Ser Val Asn Gln Pro
           35                  40                  45
   Val Phe Ser Cys Ser Ala Asp Trp Gln Arg Ile Ser Asp Phe Asn Ala
       50                  55                  60
   Lys Ser Gly Cys Asp Gly Gly Ser Ala Tyr Ser Cys Ala Asp Gln Thr
   65                  70                  75                  80
   Pro Trp Ala Val Asn Asp Asn Phe Ser Tyr Gly Phe Ala Ala Thr Ala
                   85                  90                  95
   Ile Ala Gly Gly Ser Glu Ser Ser Trp Cys Cys Ala Cys Tyr Ala Leu
               100                 105                 110
   Thr Phe Asn Ser Gly Pro Val Ala Gly Lys Thr Met Val Val Gln Ser
           115                 120                 125
   Thr Ser Thr Gly Gly Asp Leu Gly Ser Asn Gln Phe Asp Leu Ala Ile
       130                 135                 140
   Pro Gly Gly Gly Val Gly Ile Phe Asn Gly Cys Ala Ser Gln Phe Gly
   145                 150                 155                 160
   Gly Leu Pro Gly Ala Gln Tyr Gly Gly Ile Ser Asp Arg Ser Gln Cys
                   165                 170                 175
   Ser Ser Phe Pro Ala Pro Leu Gln Pro Gly Cys Gln Trp Arg Phe Asp
               180                 185                 190
   Trp Phe Gln Asn Ala Asp Asn Pro Thr Phe Thr Phe Gln Arg Val Gln
           195                 200                 205
   Cys Pro Ser Glu Leu Thr Ser Arg Thr Gly Cys Lys Arg Asp Asp Asp
       210                 215                 220
   Ala Ser Tyr Pro Val Phe Asn Pro Pro Ser Gly Gly Ser Pro Ser Thr
   225                 230                 235                 240
   Thr Ser Thr Thr Thr Ser Ser Pro Ser Gly Pro Thr Gly Asn Pro Pro
                   245                 250                 255
   Gly Gly Gly Gly Cys Thr Ala Gln Lys Trp Ala Gln Cys Gly Gly Thr
               260                 265                 270
   Gly Phe Thr Gly Cys Thr Thr Cys Val Ser Gly Thr Thr Cys Gln Val
           275                 280                 285
   Gln Asn Gln Trp Tyr Ser Gln Cys Leu
       290                 295
<210> 3
   <211> 2033
   <212> DNA
   <213> Acremonium thermophilum
<220>
   <221> CDS
   <222> (259)..(702)
<220>
   <221> Intron
   <222> (703)..(857)
<220>
   <221> CDS
   <222> (858)..(888)
<220>
   <221> Intron
   <222> (889)..(990)
<220>
   <221> CDS
   <222> (991)..(1268)
<400> 3
   ctcgaggaga ggaaccgagt ttgaaagatg ctatatatcg atagactacc ggcgtcgcct      60
   cgccctgtcc gctctcttgc attccccctg ttgatgagac gagacaaaat tcctggttag     120
   aaaagatccg tcgccgagat ttcaccagtg gtaagtcccg agaattggtc attcgacgtt     180
   caatatgagt gtcaaagcta tgggtcctaa caaagaagga agcaagagct ttaaagagac     240
   agaataacag cagcaaag atg cgt ctc cca cta ccg act ctg ctc gcc ctc       291
                       Met Arg Leu Pro Leu Pro Thr Leu Leu Ala Leu
                       1               5                   10
   ttg ccc tac tac ctc gaa gtg tcc gct cag ggg gca tcc gga acc ggc       339
   Leu Pro Tyr Tyr Leu Glu Val Ser Ala Gln Gly Ala Ser Gly Thr Gly
               15                  20                  25
   acg aca aca cgt tac tgg gat tgc tgc aag ccg agc tgc gcg tgg cct       387
   Thr Thr Thr Arg Tyr Trp Asp Cys Cys Lys Pro Ser Cys Ala Trp Pro
           30                  35                  40
   ctg aag ggc aat tcg ccc agc ccg gtg cag act tgc gac aag aat gac       435
   Leu Lys Gly Asn Ser Pro Ser Pro Val Gln Thr Cys Asp Lys Asn Asp
       45                  50                  55
   agg ccg ctg aac gat ggg gga aac acc aag tcc ggc tgc gac aac ggt      483
   Arg Pro Leu Asn Asp Gly Gly Asn Thr Lys Ser Gly Cys Asp Asn Gly
   60                  65                  70                  75
   ggc ggg gcc ttc atg tgc tca tcc cag agt ccc tgg gcc gtc aat gag      531
   Gly Gly Ala Phe Met Cys Ser Ser Gln Ser Pro Trp Ala Val Asn Glu
                   80                  85                  90
   acc acc agc tac ggc tgg gca gcc gtt cgt atc gcc ggc agt acc gag      579
   Thr Thr Ser Tyr Gly Trp Ala Ala Val Arg Ile Ala Gly Ser Thr Glu
               95                  100                 105
   tcg gcc tgg tgc tgt gcc tgc tac gag ctc acc ttc acc agt ggg ccc      627
   Ser Ala Trp Cys Cys Ala Cys Tyr Glu Leu Thr Phe Thr Ser Gly Pro
           110                 115                 120
   gtc agt gga aag aag ctc ata gtc cag gcc acg aac act ggt gga gac      675
   Val Ser Gly Lys Lys Leu Ile Val Gln Ala Thr Asn Thr Gly Gly Asp
       125                 130                 135
   ctt ggg agc aac cac ttt gac ctt gcg gtatgtgggg tttttctttc            722
   Leu Gly Ser Asn His Phe Asp Leu Ala
   140                 145
   ttcatcatcg ctctcaccat ggattcctcg gcgcaaggac caagattgag aagcgtcaat    782
   gccgggttgg acacgggagc cgggatagga acacagaggc cgtttaagac cgtcagctga    842
   cagcagagca attag att ccc gga ggt ggt gtt ggt cag tcc aat g           888
                    Ile Pro Gly Gly Gly Val Gly Gln Ser Asn
                        150                 155
   gtaggttcct tccctgaagt accggcaaca gcctgtgcgt tgctgtatac cccttttaat    948
   catagcatct tcctgctgga tacaagccaa cccattttct ag ct  tgc acg aac      1001
                                                  Ala Cys Thr Asn
                                                      160
   cag tat ggt gcg ccc ccg aac ggc tgg ggc gac agg tat ggt ggc gtg     1049
   Gln Tyr Gly Ala Pro Pro Asn Gly Trp Gly Asp Arg Tyr Gly Gly Val
           165                 170                 175
   cac tcg cgg agc gac tgc gac agc ttc ccc gcg gcg ctc aag gcc ggc     1097
   His Ser Arg Ser Asp Cys Asp Ser Phe Pro Ala Ala Leu Lys Ala Gly
       180                 185                 190
   tgc tac tgg cga ttc gac tgg ttc cag ggc gcc gac aac ccg tcc gtg     1145
   Cys Tyr Trp Arg Phe Asp Trp Phe Gln Gly Ala Asp Asn Pro Ser Val
   195                 200                 205                 210
   agc ttc aaa cag gta gcc tgc ccg gca gcc atc aca gct aag agc ggc     1193
   Ser Phe Lys Gln Val Ala Cys Pro Ala Ala Ile Thr Ala Lys Ser Gly
                   215                 220                 225
   tgt act cgc cag aac gat gcc atc aac gag act ccg act ggg ccc agc     1241
   Cys Thr Arg Gln Asn Asp Ala Ile Asn Glu Thr Pro Thr Gly Pro Ser
               230                 235                 240
   act gtg cct acc tac acc gcg tca ggc tgaaagtcgg ctggggcacc           1288
   Thr Val Pro Thr Tyr Thr Ala Ser Gly
           245                 250
   attgcccagg tgatggttgg gcatgtgtta gtctcactca ccagggacat ttgtcgcgac   1348
   ctgatcatag gcgccagggg agttgaaagg ggttgccgta cgagaagaca ttttgtcgcc   1408
   gtcttactcc cagccacttc tgtacatatt caatgacatt acatagcccg caaatatgtt   1468
   catatatcgt ggccgcccaa accgccccgg tttgcttagg ctggagctga agtggctcgc   1528
   cgatggctgt caaaggcagt cggaatattc ctcgttgctt cggcaacacg gtagctgctt   1588
   gaaccgtacc cagcattaga acaccccccg ccgagggctt gctacgtcaa tggcggggtc   1648
   tccaacccct gcgcggcaca aaaccaacca cgccctcgtc ttttatgatg tcctcgctca   1708
   aacgtcccgt gacgacactc cgctcatggt ctggtcctct gatgtagaag gggtaggtca    1768
   gccgatggtc gtcaccgtcg tcaatgcttc cctcaagctt cttgcggcct ttatcctcca   1828
   actcttccca catgagaact ccatctttcc gccttttcac aaagccactg ccctccttgt   1888
   caagggccaa aaaccaacgc cgctgatgaa tgcttccgat cgtgtttgac gcgcccgggg   1948
   tatgcatttg gttcggcgca cttttttcgt cctccagctc ccttaactcc cgttccatct    2008
   gagagggtga ctcgtctact cgact                                         2033
<210> 4
   <211> 251
   <212> PRT
   <213> Acremonium thermophilum
<400> 4
   Met Arg Leu Pro Leu Pro Thr Leu Leu Ala Leu Leu Pro Tyr Tyr Leu
   1               5                   10                  15
   Glu Val Ser Ala Gln Gly Ala Ser Gly Thr Gly Thr Thr Thr Arg Tyr
               20                  25                  30
   Trp Asp Cys Cys Lys Pro Ser Cys Ala Trp Pro Leu Lys Gly Asn Ser
           35                  40                  45
   Pro Ser Pro Val Gln Thr Cys Asp Lys Asn Asp Arg Pro Leu Asn Asp
       50                  55                  60
   Gly Gly Asn Thr Lys Ser Gly Cys Asp Asn Gly Gly Gly Ala Phe Met
   65                  70                  75                  80
   Cys Ser Ser Gln Ser Pro Trp Ala Val Asn Glu Thr Thr Ser Tyr Gly
                   85                  90                  95
   Trp Ala Ala Val Arg Ile Ala Gly Ser Thr Glu Ser Ala Trp Cys Cys
               100                 105                 110
   Ala Cys Tyr Glu Leu Thr Phe Thr Ser Gly Pro Val Ser Gly Lys Lys
           115                 120                 125
   Leu Ile Val Gln Ala Thr Asn Thr Gly Gly Asp Leu Gly Ser Asn His
       130                 135                 140
   Phe Asp Leu Ala Ile Pro Gly Gly Gly Val Gly Gln Ser Asn Ala Cys
   145                 150                 155                 160
   Thr Asn Gln Tyr Gly Ala Pro Pro Asn Gly Trp Gly Asp Arg Tyr Gly
                   165                 170                 175
   Gly Val His Ser Arg Ser Asp Cys Asp Ser Phe Pro Ala Ala Leu Lys
               180                 185                 190
   Ala Gly Cys Tyr Trp Arg Phe Asp Trp Phe Gln Gly Ala Asp Asn Pro
           195                 200                 205
   Ser Val Ser Phe Lys Gln Val Ala Cys Pro Ala Ala Ile Thr Ala Lys
       210                 215                 220
   Ser Gly Cys Thr Arg Gln Asn Asp Ala Ile Asn Glu Thr Pro Thr Gly
   225                 230                 235                 240
   Pro Ser Thr Val Pro Thr Tyr Thr Ala Ser Gly
                   245                 250
<210> 5
   <211> 17
   <212> PRT
   <213> Acremonium thermophilum
<400> 5
   Gln Ser Cys Ser Ser Phe Pro Ala Pro Leu Lys Pro Gly Cys Gln Trp
   1               5                   10                  15
   Arg
<210> 6
   <211> 13
   <212> PRT
   <213> Acremonium thermophilum
<400> 6
   Tyr Ala Leu Thr Phe Asn Ser Gly Pro Val Ala Gly Lys
   1 5 10
<210> 7
   <211> 10
   <212> PRT
   <213> Acremonium thermophilum
<400> 7
   Val Gln Cys Pro Ser Glu Leu Thr Ser Arg
   1 5 10
<210> 8
   <211> 13
   <212> PRT
   <213> Acremonium thermophilum
<400> 8
   Asn Gln Pro Val Phe Ser Cys Ser Ala Asp Trp Gln Arg
   1 5 10
<210> 9
   <211> 14
   <212> PRT
   <213> Acremonium thermophilum
<400> 9
   Tyr Trp Asp Cys Cys Lys Pro Ser Cys Gly Trp Pro Gly Lys
   1 5 10
<210> 10
   <211> 4
   <212> PRT
   <213> Acremonium thermophilum
<400> 10
   Pro Thr Phe Thr
   1
<210> 11
   <211> 20
   <212> DNA
   <213> Artificial Sequence
<220>
   <223> primer
<220>
   <221> misc_feature
   <222> (3)..(3)
   <223> y means c or t
<220>
   <221> misc_feature
   <222> (9)..(9)
   <223> y means c or t
<220>
   <221> misc_feature
   <222> (12)..(12)
   <223> y means c or t
<220>
   <221> misc_feature
   <222> (15)..(15)
   <223> r means a or g
<400> 11
   taytgggayt gytgyaarcc 20
<210> 12
   <211> 21
   <212> DNA
   <213> Artificial Sequence
<220>
   <223> primer
<220>
   <221> misc_feature
   <222> (1)..(1)
   <223> r means a or g
<220>
   <221> misc_feature
   <222> (4)..(4)
   <223> r means a or g
<220>
   <221> misc_feature
   <222> (7)..(7)
   <223> n means a, t, c or g
<220>
   <221> misc_feature
   <222> (10)..(10)
   <223> r means a or g
<220>
   <221> misc_feature
   <222> (13)..(13)
   <223> y means c or t
<220>
   <221> misc_feature
   <222> (16)..(16)
   <223> r means a or g
<400> 12
   rttrtcngcr ttytgraacc a 21
<210> 13
   <211> 7
   <212> PRT
   <213> Melanocarpus albomyces
<400> 13
   Trp Phe Gln Asn Ala Asp Asn
   1               5
<210> 14
   <211> 636
   <212> DNA
   <213> Acremonium thermophilum
<400> 14
   tactgggatt gttgcaagcc gtcctgcggc tggccgggaa aggcctcggt gaaccagccc     60
   gtcttctcgt gctcggccga ctggcagcgc atcagcgact tcaacgcgaa gtcgggctgc    120
   gacggaggct ccgcctactc gtgcgccgac cagacgccct gggcggtcaa cgacaacttc    180
   tcgtacggct tcgcagccac ggccatcgcc ggcggctccg agtccagctg gtgctgcgcc    240
   tgctatgcgt gagttctctg caagccgctt cccacccccg ctttctgtgc aggccgcttc    300
   ccccctaccc acccacttcc ccccccccgc ctctgtgatc gggcatccga gctaagttgc    360
   gtgtcgtcca gactcacctt caactcgggc cccgtcgcgg gcaagaccat ggtggtgcag    420
   tcgaccagca ccggcggcga cctgggcagc aaccagttcg acctcgccat ccccggcggc    480
   ggcgtgggca tcttcaacgg ctgcgcctcc cagttcggcg gcctccccgg cgcccagtac    540
   ggcggcatca gcgaccgcag ccagtgctcg tccttccccg cgccgctcca gccgggctgc    600
   cagtggcgct tcgactggtt ccagaacgcg gataat                              636
<210> 15
   <211> 786
   <212> DNA
   <213> Acremonium thermophilum
<400> 15
   tactgggatt gttgcaagcc gagctgcgcg tggcctctga agggcaattc gcccagcccg     60
   gtgcagactt gcgacaagaa tgacaggccg ctgaacgatg ggggaaacac caagtccggc    120
   tgcgacaacg gtggcggggc cttcatgtgc tcatcccaga gtccctgggc cgtcaatgag    180
   accaccagct acggctgggc agccgttcgt atcgccggca gtaccgagtc ggcctggtgc    240
   tgtgcctgct acgagctcac cttcaccagt gggcccgtca gtggaaagaa gctcatagtc    300
   caggccacga acactggtgg agaccttggg agcaaccact ttgaccttgc ggtatgtggg    360
   gtttttcttt cttcatcatc gctctcacca tggattcctc ggcgcaagga ccaagattga    420
   gaagcgtcaa tgccgggttg gacacgggag ccgggatagg aacacagagg ccgtttaaga    480
   ccgtcagctg acagcaggag caattagatt cccggaggtg gtgttggtca gttcaatggt    540
   aggttccttc cctgaagtac cggcaacagc ctgtgcgttg ctgtataccc cttttaatca    600
   tagcatcttc ctgctggata caagccaacc cattttctag cttgcacgaa ccagtatggt    660
   gcgcccccga acggctgggg cgacaggtat ggtggcgtgc actcgcggag cgactgcgac    720
   agcttccccg cggcgctcaa ggccggctgc tactggcgat tcgactggtt tcaaaacgcc    780
   gacaac                                                                786
<210> 16
   <211> 994
   <212> DNA
   <213> Acremonium thermophilum
<220>
   <221> CDS
   <222> (13)..(95)
<220>
   <221> Intron
   <222> (96)..(154)
<220>
   <221> CDS
   <222> (155)..(403)
<220>
   <221> Intron
   <222> (404)..(526)
<220>
   <221> CDS
   <222> (527)..(986)
<400> 16
   ggactgcgca tc atg cgc tcc tca ccc ttt ctc cgc gca gct ctg gct gcc      51
                 Met Arg Ser Ser Pro Phe Leu Arg Ala Ala Leu Ala Ala
                 1               5                   10
   gct ctg cct ctg agc gcc cat gcc ctc gac gga aag tcg acg ag             95
   Ala Leu Pro Leu Ser Ala His Ala Leu Asp Gly Lys Ser Thr Arg
       15                  20                  25
   gtatgccaat cctcgtacct ctgccctctg tagaaacaag tgaccgactg caaagacag      154
   a tac tgg gac tgc tgc aag ccg tcc tgc ggc tgg gcc gga aag gcc tcg    203
     Tyr Trp Asp Cys Cys Lys Pro Ser Cys Gly Trp Ala Gly Lys Ala Ser
         30                  35                  40
   gtg aac cag ccc gtc ttc tcg tgc tcg gcc gac tgg cag cgc atc agc      251
   Val Asn Gln Pro Val Phe Ser Cys Ser Ala Asp Trp Gln Arg Ile Ser
   45                  50                  55                  60
   gac ttc aac gcg aag tcg ggc tgc gac gga ggc tcc gcc tac tcg tgc       299
   Asp Phe Asn Ala Lys Ser Gly Cys Asp Gly Gly Ser Ala Tyr Ser Cys
                   65                  70                  75
   gcc gac cag acg ccc tgg gcg gtc aac gac aac ttc tcg tac ggc ttc       347
   Ala Asp Gln Thr Pro Trp Ala Val Asn Asp Asn Phe Ser Tyr Gly Phe
               80                  85                  90
   gca gcc acg gcc atc gcc ggc ggc tcc gag tcc agc tgg tgc tgc gcc       395
   Ala Ala Thr Ala Ile Ala Gly Gly Ser Glu Ser Ser Trp Cys Cys Ala
           95                  100                 105
   tgc tat gc  gtgagttctc tgcaagccgc ttcccacccc cgctttctgt               443
   Cys Tyr Ala
       110
   gcaggccgct tcccccctac ccacccactt cccccccccc gcctctgtga tcgggcatcc     503
   gagctaagtt gcgtgtcgtc cag a ctc acc ttc aac tcg ggc ccc gtc gcg       554
                               Leu Thr Phe Asn Ser Gly Pro Val Ala
                                           115                 120
   ggc aag acc atg gtg gtg cag tcg acc agc acc ggc ggc gac ctg ggc       602
   Gly Lys Thr Met Val Val Gln Ser Thr Ser Thr Gly Gly Asp Leu Gly
                   125                 130                 135
   agc aac cag ttc gac ctc gcc atc ccc ggc ggc ggc gtg ggc atc ttc       650
   Ser Asn Gln Phe Asp Leu Ala Ile Pro Gly Gly Gly Val Gly Ile Phe
               140                 145                 150
   aac ggc tgc gcc tcc cag ttc ggc ggc ctc ccc ggc gcc cag tac ggc       698
   Asn Gly Cys Ala Ser Gln Phe Gly Gly Leu Pro Gly Ala Gln Tyr Gly
           155                 160                 165
   ggc atc agc gac cgc agc cag tgc tcg tcc ttc ccc gcg ccg ctc cag       746
   Gly Ile Ser Asp Arg Ser Gln Cys Ser Ser Phe Pro Ala Pro Leu Gln
       170                 175                 180
   ccg ggc tgc cag tgg cgc ttc gac tgg ttc cag aac gcc gac aac ccc       794
   Pro Gly Cys Gln Trp Arg Phe Asp Trp Phe Gln Asn Ala Asp Asn Pro
   185                 190                 195                 200
   acc ttc acc ttc cag cgc gtg cag tgc ccg tcc gag ctc acg tcc cgc       842
   Thr Phe Thr Phe Gln Arg Val Gln Cys Pro Ser Glu Leu Thr Ser Arg
                   205                 210                 215
   acg ggc tgt aag cgc gac gac gac gcc agc tat ccc gtc ttc aac ccg       890
   Thr Gly Cys Lys Arg Asp Asp Asp Ala Ser Tyr Pro Val Phe Asn Pro
               220                 225                 230
   cct agc ggt ggc tcc ccc agc acc acc agc acc acc acc agc tcc ccg       938
   Pro Ser Gly Gly Ser Pro Ser Thr Thr Ser Thr Thr Thr Ser Ser Pro
           235                 240                 245
   tcc ggt ccc acg ggc aac cct cct gga ggc ggt ggc tgc act gcc cag       986
   Ser Gly Pro Thr Gly Asn Pro Pro Gly Gly Gly Gly Cys Thr Ala Gln
       250                 255                 260
   tgactgca                                                              994
<210> 17
   <211> 264
   <212> PRT
   <213> Acremonium thermophilum
<400> 17
   Met Arg Ser Ser Pro Phe Leu Arg Ala Ala Leu Ala Ala Ala Leu Pro
   1               5                   10                  15
   Leu Ser Ala His Ala Leu Asp Gly Lys Ser Thr Arg Tyr Trp Asp Cys
               20                  25                  30
   Cys Lys Pro Ser Cys Gly Trp Ala Gly Lys Ala Ser Val Asn Gln Pro
           35                  40                  45
   Val Phe Ser Cys Ser Ala Asp Trp Gln Arg Ile Ser Asp Phe Asn Ala
       50                  55                  60
   Lys Ser Gly Cys Asp Gly Gly Ser Ala Tyr Ser Cys Ala Asp Gln Thr
   65                  70                  75                  80
   Pro Trp Ala Val Asn Asp Asn Phe Ser Tyr Gly Phe Ala Ala Thr Ala
                   85                  90                  95
   Ile Ala Gly Gly Ser Glu Ser Ser Trp Cys Cys Ala Cys Tyr Ala Leu
               100                 105                 110
   Thr Phe Asn Ser Gly Pro Val Ala Gly Lys Thr Met Val Val Gln Ser
           115                 120                 125
   Thr Ser Thr Gly Gly Asp Leu Gly Ser Asn Gln Phe Asp Leu Ala Ile
       130                 135                 140
   Pro Gly Gly Gly Val Gly Ile Phe Asn Gly Cys Ala Ser Gln Phe Gly
   145                 150                 155                 160
   Gly Leu Pro Gly Ala Gln Tyr Gly Gly Ile Ser Asp Arg Ser Gln Cys
                   165                 170                 175
   Ser Ser Phe Pro Ala Pro Leu Gln Pro Gly Cys Gln Trp Arg Phe Asp
               180                 185                 190
   Trp Phe Gln Asn Ala Asp Asn Pro Thr Phe Thr Phe Gln Arg Val Gln
           195                 200                 205
   Cys Pro Ser Glu Leu Thr Ser Arg Thr Gly Cys Lys Arg Asp Asp Asp
       210                 215                 220
   Ala Ser Tyr Pro Val Phe Asn Pro Pro Ser Gly Gly Ser Pro Ser Thr
   225                 230                 235                 240
   Thr Ser Thr Thr Thr Ser Ser Pro Ser Gly Pro Thr Gly Asn Pro Pro
                   245                 250                 255
   Gly Gly Gly Gly Cys Thr Ala Gln
               260
<210> 18
   <211> 907
   <212> DNA
   <213> Acremonium thermophilum
<220>
   <221> CDS
   <222> (13)..(95)
<220>
   <221> Intron
   <222> (96)..(154)
<220>
   <221> CDS
   <222> (155)..(403)
<220>
   <221> Intron
   <222> (404)..(526)
<220>
   <221> CDS
   <222> (527)..(899)
<400> 18
   ggactgcgca tc atg cgc tcc tca ccc ttt ctc cgc gca gct ctg gct gcc     51
                 Met Arg Ser Ser Pro Phe Leu Arg Ala Ala Leu Ala Ala
                 1               5                   10
   gct ctg cct ctg agc gcc cat gcc ctc gac gga aag tcg acg ag            95
   Ala Leu Pro Leu Ser Ala His Ala Leu Asp Gly Lys Ser Thr Arg
       15                  20                  25
   gtatgccaat cctcgtacct ctgccctctg tagaaacaag tgaccgactg caaagacag     154
   a tac tgg gac tgc tgc aag ccg tcc tgc ggc tgg gcc gga aag gcc tcg    203
     Tyr Trp Asp Cys Cys Lys Pro Ser Cys Gly Trp Ala Gly Lys Ala Ser
         30                  35                  40
   gtg aac cag ccc gtc ttc tcg tgc tcg gcc gac tgg cag cgc atc agc      251
   Val Asn Gln Pro Val Phe Ser Cys Ser Ala Asp Trp Gln Arg Ile Ser
   45                  50                  55                  60
   gac ttc aac gcg aag tcg ggc tgc gac gga ggc tcc gcc tac tcg tgc      299
   Asp Phe Asn Ala Lys Ser Gly Cys Asp Gly Gly Ser Ala Tyr Ser Cys
                   65                  70                  75
   gcc gac cag acg ccc tgg gcg gtc aac gac aac ttc tcg tac ggc ttc      347
   Ala Asp Gln Thr Pro Trp Ala Val Asn Asp Asn Phe Ser Tyr Gly Phe
               80                  85                  90
   gca gcc acg gcc atc gcc ggc ggc tcc gag tcc agc tgg tgc tgc gcc      395
   Ala Ala Thr Ala Ile Ala Gly Gly Ser Glu Ser Ser Trp Cys Cys Ala
           95                  100                 105
   tgc tat gc  gtgagttctc tgcaagccgc ttcccacccc cgctttctgt              443
   Cys Tyr Ala
       110
   gcaggccgct tcccccctac ccacccactt cccccccccc gcctctgtga tcgggcatcc    503
   gagctaagtt gcgtgtcgtc cag a ctc acc ttc aac tcg ggc ccc gtc gcg      554
                               Leu Thr Phe Asn Ser Gly Pro Val Ala
                                           115                 120
   ggc aag acc atg gtg gtg cag tcg acc agc acc ggc ggc gac ctg ggc      602
   Gly Lys Thr Met Val Val Gln Ser Thr Ser Thr Gly Gly Asp Leu Gly
                   125                 130                 135
   agc aac cag ttc gac ctc gcc atc ccc ggc ggc ggc gtg ggc atc ttc      650
   Ser Asn Gln Phe Asp Leu Ala Ile Pro Gly Gly Gly Val Gly Ile Phe
               140                 145                 150
   aac ggc tgc gcc tcc cag ttc ggc ggc ctc ccc ggc gcc cag tac ggc      698
   Asn Gly Cys Ala Ser Gln Phe Gly Gly Leu Pro Gly Ala Gln Tyr Gly
           155                 160                 165
   ggc atc agc gac cgc agc cag tgc tcg tcc ttc ccc gcg ccg ctc cag      746
   Gly Ile Ser Asp Arg Ser Gln Cys Ser Ser Phe Pro Ala Pro Leu Gln
       170                 175                 180
   ccg ggc tgc cag tgg cgc ttc gac tgg ttc cag aac gcc gac aac ccc      794
   Pro Gly Cys Gln Trp Arg Phe Asp Trp Phe Gln Asn Ala Asp Asn Pro
   185                 190                 195                 200
   acc ttc acc ttc cag cgc gtg cag tgc ccg tcc gag ctc acg tcc cgc      842
   Thr Phe Thr Phe Gln Arg Val Gln Cys Pro Ser Glu Leu Thr Ser Arg
                   205                 210                 215
   acg ggc tgt aag cgc gac gac gac gcc agc tat ccc gtc ttc aac ccg      890
   Thr Gly Cys Lys Arg Asp Asp Asp Ala Ser Tyr Pro Val Phe Asn Pro
               220                 225                 230
   cct agc ggt tgactgca                                                 907
   Pro Ser Gly
           235
<210> 19
   <211> 235
   <212> PRT
   <213> Acremonium thermophilum
<400> 19
   Met Arg Ser Ser Pro Phe Leu Arg Ala Ala Leu Ala Ala Ala Leu Pro
   1               5                   10                  15
   Leu Ser Ala His Ala Leu Asp Gly Lys Ser Thr Arg Tyr Trp Asp Cys
               20                  25                  30
   Cys Lys Pro Ser Cys Gly Trp Ala Gly Lys Ala Ser Val Asn Gln Pro
           35                  40                  45
   Val Phe Ser Cys Ser Ala Asp Trp Gln Arg Ile Ser Asp Phe Asn Ala
       50                  55                  60
   Lys Ser Gly Cys Asp Gly Gly Ser Ala Tyr Ser Cys Ala Asp Gln Thr
   65                  70                  75                  80
   Pro Trp Ala Val Asn Asp Asn Phe Ser Tyr Gly Phe Ala Ala Thr Ala
                   85                  90                  95
   Ile Ala Gly Gly Ser Glu Ser Ser Trp Cys Cys Ala Cys Tyr Ala Leu
               100                 105                 110
   Thr Phe Asn Ser Gly Pro Val Ala Gly Lys Thr Met Val Val Gln Ser
           115                 120                 125
   Thr Ser Thr Gly Gly Asp Leu Gly Ser Asn Gln Phe Asp Leu Ala Ile
       130                 135                 140
   Pro Gly Gly Gly Val Gly Ile Phe Asn Gly Cys Ala Ser Gln Phe Gly
   145                 150                 155                 160
   Gly Leu Pro Gly Ala Gln Tyr Gly Gly Ile Ser Asp Arg Ser Gln Cys
                   165                 170                 175
   Ser Ser Phe Pro Ala Pro Leu Gln Pro Gly Cys Gln Trp Arg Phe Asp
               180                 185                 190
   Trp Phe Gln Asn Ala Asp Asn Pro Thr Phe Thr Phe Gln Arg Val Gln
           195                 200                 205
   Cys Pro Ser Glu Leu Thr Ser Arg Thr Gly Cys Lys Arg Asp Asp Asp
       210                 215                 220
   Ala Ser Tyr Pro Val Phe Asn Pro Pro Ser Gly
   225                 230                 235
<210> 20
   <211> 1175
   <212> DNA
   <213> Acremonium thermophilum
<220>
   <221> CDS
   <222> (1)..(444)
<220>
   <221> Intron
   <222> (445)..(599)
<220>
   <221> CDS
   <222> (600)..(630)
<220>
   <221> Intron
   <222> (631)..(732)
<220>
   <221> CDS
   <222> (733)..(1172)
<400> 20
   atg cgt ctc cca cta ccg act ctg ctc gcc ctc ttg ccc tac tac ctc       48
   Met Arg Leu Pro Leu Pro Thr Leu Leu Ala Leu Leu Pro Tyr Tyr Leu
   1               5                   10                  15
   gaa gtg tcc gct cag ggg gca tcc gga acc ggc acg aca aca cgt tac       96
   Glu Val Ser Ala Gln Gly Ala Ser Gly Thr Gly Thr Thr Thr Arg Tyr
               20                  25                  30
   tgg gat tgc tgc aag ccg agc tgc gcg tgg cct ctg aag ggc aat tcg      144
   Trp Asp Cys Cys Lys Pro Ser Cys Ala Trp Pro Leu Lys Gly Asn Ser
           35                  40                  45
   ccc agc ccg gtg cag act tgc gac aag aat gac agg ccg ctg aac gat      192
   Pro Ser Pro Val Gln Thr Cys Asp Lys Asn Asp Arg Pro Leu Asn Asp
       50                  55                  60
   ggg gga aac acc aag tcc ggc tgc gac aac ggt ggc ggg gcc ttc atg      240
   Gly Gly Asn Thr Lys Ser Gly Cys Asp Asn Gly Gly Gly Ala Phe Met
   65                  70                  75                  80
   tgc tca tcc cag agt ccc tgg gcc gtc aat gag acc acc agc tac ggc      288
   Cys Ser Ser Gln Ser Pro Trp Ala Val Asn Glu Thr Thr Ser Tyr Gly
                   85                  90                  95
   tgg gca gcc gtt cgt atc gcc ggc agt acc gag tcg gcc tgg tgc tgt      336
   Trp Ala Ala Val Arg Ile Ala Gly Ser Thr Glu Ser Ala Trp Cys Cys
               100                 105                 110
   gcc tgc tac gag ctc acc ttc acc agt ggg ccc gtc agt gga aag aag      384
   Ala Cys Tyr Glu Leu Thr Phe Thr Ser Gly Pro Val Ser Gly Lys Lys
           115                 120                 125
   ctc ata gtc cag gcc acg aac act ggt gga gac ctt ggg agc aac cac      432
   Leu Ile Val Gln Ala Thr Asn Thr Gly Gly Asp Leu Gly Ser Asn His
       130                 135                 140
   ttt gac ctt gcg gtatgtgggg tttttctttc ttcatcatcg ctctcaccat          484
   Phe Asp Leu Ala
   145
   ggattcctcg gcgcaaggac caagattgag aagcgtcaat gccgggttgg acacgggagc    544
   cgggatagga acacagaggc cgtttaagac cgtcagctga cagcagagca attag att     602
                                                                Ile
   ccc gga ggt ggt gtt ggt cag tcc aat g gtaggttcct tccctgaagt          650
   Pro Gly Gly Gly Val Gly Gln Ser Asn
   150                 155
   accggcaaca gcctgtgcgt tgctgtatac cccttttaat catagcatct tcctgctgga     710
   tacaagccaa cccattttct ag ct  tgc acg aac cag tat ggt gcg ccc ccg     761
                            Ala Cys Thr Asn Gln Tyr Gly Ala Pro Pro
                                160                 165
   aac ggc tgg ggc gac agg tat ggt ggc gtg cac tcg cgg agc gac tgc      809
   Asn Gly Trp Gly Asp Arg Tyr Gly Gly Val His Ser Arg Ser Asp Cys
       170                 175                 180
   gac agc ttc ccc gcg gcg ctc aag gcc ggc tgc tac tgg cga ttc gac      857
   Asp Ser Phe Pro Ala Ala Leu Lys Ala Gly Cys Tyr Trp Arg Phe Asp
   185                 190                 195                 200
   tgg ttc cag ggc gcc gac aac ccg tcc gtg agc ttc aaa cag gta gcc      905
   Trp Phe Gln Gly Ala Asp Asn Pro Ser Val Ser Phe Lys Gln Val Ala
                   205                 210                 215
   tgc ccg gca gcc atc aca gct aag agc ggc tgt act cgc cag aac gat      953
   Cys Pro Ala Ala Ile Thr Ala Lys Ser Gly Cys Thr Arg Gln Asn Asp
               220                 225                 230
   gcc atc aac gag act ccg act ggg ccc agc ggt ggc tcc ccc agc acc     1001
   Ala Ile Asn Glu Thr Pro Thr Gly Pro Ser Gly Gly Ser Pro Ser Thr
           235                 240                 245
   acc agc acc acc acc agc tcc ccg tcc ggt ccc acg ggc aac cct cct     1049
   Thr Ser Thr Thr Thr Ser Ser Pro Ser Gly Pro Thr Gly Asn Pro Pro
       250                 255                 260
   gga ggc ggt ggc tgc act gcc cag aag tgg gcc cag tgc ggc ggc act     1097
   Gly Gly Gly Gly Cys Thr Ala Gln Lys Trp Ala Gln Cys Gly Gly Thr
   265                 270                 275                 280
   ggc ttc acg ggc tgc acc acc tgc gtc tcg ggc acc acc tgc cag gtg     1145
   Gly Phe Thr Gly Cys Thr Thr Cys Val Ser Gly Thr Thr Cys Gln Val
                   285                 290                 295
   cag aac cag tgg tat tcc cag tgt ctg tga                             1175
   Gln Asn Gln Trp Tyr Ser Gln Cys Leu
               300                 305
<210> 21
   <211> 305
   <212> PRT
   <213> Acremonium thermophilum
<400> 21
   Met Arg Leu Pro Leu Pro Thr Leu Leu Ala Leu Leu Pro Tyr Tyr Leu
   1               5                   10                  15
   Glu Val Ser Ala Gln Gly Ala Ser Gly Thr Gly Thr Thr Thr Arg Tyr
               20                  25                  30
   Trp Asp Cys Cys Lys Pro Ser Cys Ala Trp Pro Leu Lys Gly Asn Ser
           35                  40                  45
   Pro Ser Pro Val Gln Thr Cys Asp Lys Asn Asp Arg Pro Leu Asn Asp
       50                  55                  60
   Gly Gly Asn Thr Lys Ser Gly Cys Asp Asn Gly Gly Gly Ala Phe Met
   65                  70                  75                  80
   Cys Ser Ser Gln Ser Pro Trp Ala Val Asn Glu Thr Thr Ser Tyr Gly
                   85                  90                  95
   Trp Ala Ala Val Arg Ile Ala Gly Ser Thr Glu Ser Ala Trp Cys Cys
               100                 105                 110
   Ala Cys Tyr Glu Leu Thr Phe Thr Ser Gly Pro Val Ser Gly Lys Lys
           115                 120                 125
   Leu Ile Val Gln Ala Thr Asn Thr Gly Gly Asp Leu Gly Ser Asn His
       130                 135                 140
   Phe Asp Leu Ala Ile Pro Gly Gly Gly Val Gly Gln Ser Asn Ala Cys
   145                 150                 155                 160
   Thr Asn Gln Tyr Gly Ala Pro Pro Asn Gly Trp Gly Asp Arg Tyr Gly
                   165                 170                 175
   Gly Val His Ser Arg Ser Asp Cys Asp Ser Phe Pro Ala Ala Leu Lys
               180                 185                 190
   Ala Gly Cys Tyr Trp Arg Phe Asp Trp Phe Gln Gly Ala Asp Asn Pro
           195                 200                 205
   Ser Val Ser Phe Lys Gln Val Ala Cys Pro Ala Ala Ile Thr Ala Lys
       210                 215                 220
   Ser Gly Cys Thr Arg Gln Asn Asp Ala Ile Asn Glu Thr Pro Thr Gly
   225                 230                 235                 240
   Pro Ser Gly Gly Ser Pro Ser Thr Thr Ser Thr Thr Thr Ser Ser Pro
                   245                 250                 255
   Ser Gly Pro Thr Gly Asn Pro Pro Gly Gly Gly Gly Cys Thr Ala Gln
               260                 265                 270
   Lys Trp Ala Gln Cys Gly Gly Thr Gly Phe Thr Gly Cys Thr Thr Cys
           275                 280                 285
   Val Ser Gly Thr Thr Cys Gln Val Gln Asn Gln Trp Tyr Ser Gln Cys
       290                 295                 300
   Leu
   305
<210> 22
   <211> 89
   <212> DNA
   <213> Acremonium thermophilum
<400> 22
   attaaccgcg gactgcgcat catgtatcgg aagttggccg tcatctcggc cttcttggcc     60
   acagctcgtg ccctcgacgg aaagtcgac                                       89
<210> 23
   <211> 20
   <212> DNA
   <213> Acremonium thermophilum
<400> 23
   tcgactgcac caccatggtc 20
<210> 24
   <211> 68
   <212> PRT
   <213> Trichoderma reesei
<400> 24
   Gly Asn Pro Ser Gly Gly Asn Pro Pro Gly Gly Asn Pro Pro Gly Thr
   1               5                   10                  15
   Thr Thr Thr Arg Arg Pro Ala Thr Thr Thr Gly Ser Ser Pro Gly Pro
               20                  25                  30
   Thr Gln Ser His Tyr Gly Gln Cys Gly Gly Ile Gly Tyr Ser Gly Pro
           35                  40                  45
   Thr Val Cys Ala Ser Gly Thr Thr Cys Gln Val Leu Asn Pro Tyr Tyr
       50                  55                  60
   Ser Gln Cys Leu
   65
<210> 25
   <211> 70
   <212> PRT
   <213> Acremonium thermophilum
<400> 25
   Gly Gly Asn Pro Pro Pro Val Thr Thr Thr Thr Thr Thr Thr Thr Thr
   1               5                   10                  15
   Ser Lys Pro Ser Gln Pro Thr Thr Thr Thr Thr Thr Thr Ser Pro Gln
               20                  25                  30
   Gly Pro Gln Gln Thr His Trp Gly Gln Cys Gly Gly Ile Gly Trp Thr
           35                  40                  45
   Gly Pro Gln Ser Cys Gln Ser Pro Trp Thr Cys Gln Lys Gln Asn Asp
       50                  55                  60
   Trp Tyr Ser Gln Cys Leu
   65                  70
<210> 26
   <211> 81
   <212> PRT
   <213> Chaetomium thermophilum
<400> 26
   Val Pro Gly Leu Asp Gly Ser Asn Pro Gly Asn Pro Thr Thr Thr Val
   1               5                   10                  15
   Val Pro Pro Ala Ser Thr Ser Thr Ser Arg Pro Thr Ser Ser Thr Ser
               20                  25                  30
   Ser Pro Val Ser Thr Pro Thr Gly Gln Pro Gly Gly Cys Thr Thr Gln
           35                  40                  45
   Lys Trp Gly Gln Cys Gly Gly Ile Gly Tyr Thr Gly Cys Thr Asn Cys
       50                  55                  60
   Val Ala Gly Thr Thr Cys Thr Gln Leu Asn Pro Trp Tyr Ser Gln Cys
   65                  70                  75                  80
   Leu
<210> 27
   <211> 1675
   <212> DNA
   <213> Trichoderma reesei
<220>
   <221> Intron
   <222> (462)..(528)
<220>
   <221> Intron
   <222> (1226)..(1288)
<400> 27
   atgtatcgga agttggccgt catctcggcc ttcttggcca cagctcgtgc tcagtcggcc     60
   tgcactctcc aatcggagac tcacccgcct ctgacatggc agaaatgctc gtctggtggc    120
   acttgcactc aacagacagg ctccgtggtc atcgacgcca actggcgctg gactcacgct    180
   acgaacagca gcacgaactg ctacgatggc aacacttgga gctcgaccct atgtcctgac    240
   aacgagacct gcgcgaagaa ctgctgtctg gacggtgccg cctacgcgtc cacgtacgga    300
   gttaccacga gcggtaacag cctctccatt ggctttgtca cccagtctgc gcagaagaac    360
   gttggcgctc gcctttacct tatgggcagc gacacgacct accaggaatt caccctgctt    420
   ggcaacgagt tctctttcga tgttgatgtt tcgcagctgc cgtaagtgac ttaccatgaa    480
   cccctgacgt atcttcttgt gggctcccag ctgactggcc aatttaaggt gcggcttgaa    540
   cggagctctc tacttcgtgt ccatggacgc ggatggtggc gtgagcaagt atcccaccaa    600
   caccgctggc gccaagtacg gcacggggta ctgtgacagc cagtgtcccc gcgatctgaa    660
   gttcatcaat ggccaggcca acgttgaggg ctgggagccg tcatccaaca acgcaaacac    720
   gggcattgga ggacacggaa gctgctgctc tgagatggat atctgggagg ccaactccat    780
   ctccgaggct cttacccccc acccttgcac gactgtcggc caggagatct gcgagggtga    840
   tgggtgcggc ggaacttact ccgataacag atatggcggc acttgcgatc ccgatggctg    900
   cgactggaac ccataccgcc tgggcaacac cagcttctac ggccctggct caagctttac    960
   cctcgatacc accaagaaat tgaccgttgt cacccagtcc gagacgtcgg gtgccatcaa   1020
   ccgatactat gtccagaatg gcgtcacttt ccagcagccc aacgccgagc ttggtagtta   1080
   ctctggcaac gagctcaacg atgattactg cacagctgag gaggcagaat tcggcggatc   1140
   ctctttctca gacaagggcg gcctgactca gttcaagaag gctacctctg gcggcatggt   1200
   tctggtcatg agtctgtggg atgatgtgag tttgatggac aaacatgcgc gttgacaaag   1260
   agtcaagcag ctgactgaga tgttacagta ctacgccaac atgctgtggc tggactccac   1320
   ctacccgaca aacgagacct cctccacacc cggtgccgtg cgcggaagct gctccaccag   1380
   ctccggtgtc cctgctcagg tcgaatctca gtctcccaac gccaaggtca ccttctccaa   1440
   catcaagttc ggacccattg gcagcaccgg caaccctagc ggcggcaacc ctcccggcgg   1500
   aaacccgcct ggcaccacca ccacccgccg cccagccact accactggaa gctctcccgg   1560
   acctacccag tctcactacg gccagtgcgg cggtattggc tacagcggcc ccacggtctg   1620
   cgccagcggc acaacttgcc aggtcctgaa cccttactac tctcagtgcc tgtaa        1675
<210> 28
   <211> 1471
   <212> DNA
   <213> Acremonium thermophilum
<220>
   <221> Intron
   <222> (338)..(472)
<220>
   <221> Intron
   <222> (699)..(783)
<400> 28
   atgcgcgcca agcaactcct ggcggccggc ctgctggccc ccgcgtccgt ctcggcccag      60
   ctcaacagcc tcgccgtggc ggctggcctc aagtacttcg gcacggccgt gcgggaggcc     120
   aacgtcaacg gcgacgccac ctacatgtcg tacgtcaaca acaagtccga gttcggccag     180
   gtgacgcccg agaacggcca gaagtgggat tccaccgagc ccagccaggg ccagttcagc     240
   tacagccagg gcgacatcgt ccccggcgtc gcgaagaaga acggccaggt gctgcgctgc     300
   cacaccctgg tgtggtacag ccagctcccc agctggggtc agtgactctc tctttctctc     360
   tgtctttctc tttgtctttc tctctttctc tctctctctc tctctctctc tctctctctc     420
   tctcccatcc agcatcgact gctgatcttg ctgaccagaa gctcgtgtgc agtgtcatcc     480
   ggaagttgga cccgcgcgac gcttcagtcc gtcatcgaga cgcacatctc gaacgtgatg     540
   ggccactaca agggccagtg ctacgcctgg gacgtggtca acgaggccat caacgacgac     600
   ggcacgtggc ggaccagcgt cttctacaac accttcaaca ccgactacct ggccattgcc     660
   ttcaacgccg cgaagaaggc cgatgcgggc gcgaagctgt aggtgtcggc ctttacgttg     720
   ccgcagcgca cctccgcgac atgagcccca gagcgcgtgg ctaatagttc ctcacgcacg     780
   caggtactac aacgactaca atctcgagta caacggcgcc aagaccaaca cggccgtgca     840
   gctggtgcag atcgtgcagc aggccggcgc gcccatcgac ggggtgggct tccagggcca     900
   cctgatcgtg gggtcaacgc cgtcgcgcag ctccctggcc acggcgctga agcgcttcac     960
   ggcgcttggc ctggaggtgg cgtacacgga gctggacatc cggcactcga gcctgccgcc    1020
   gtcgtcggcg gcgctggcga cgcagggcaa cgacttcgcc agcgtggtgg gctcgtgcct    1080
   cgacgtggcg ggctgcgtgg gcatcaccat ctgggggttc acggacaagt acagctgggt    1140
   gcccgacacg ttccccggct cgggcgcggc gctgctgtac gacgcgaact acagcaagaa    1200
   gccggcgtgg acgtcggtct cgtcggtgct ggcggccaag gcgacgaacc cgcccggcgg    1260
   cgggaaccca ccccccgtca ccaccacgac cacgaccacg accacgtcga agccgtcgca    1320
   gcccaccacc acgaccacga ccaccagccc gcagggtccg cagcagacgc actggggcca    1380
   gtgcggcggg atcggctgga cggggccgca gtcgtgccag agcccgtgga cgtgccagaa    1440
   gcagaacgac tggtactctc agtgcctgtg a                                   1471
<210> 29
   <211> 1663
   <212> DNA
   <213> Chaetomium thermophilum
<220>
   <221> Intron
   <222> (1591)..(1654)
<400> 29
   atgatgtata agaagttcgc cgctctcgcc gccctcgtgg ctggcgcctc cgcccagcag      60
   gcttgctccc tcaccgctga gaaccaccct agcctcacct ggaagcgctg cacctctggc     120
   ggcagctgct cgaccgtgaa cggcgccgtc accatcgatg ccaactggcg ctggactcac     180
   accgtctccg gctcgaccaa ctgctacacc ggcaaccagt gggatacctc cctctgcact     240
   gatggcaaga gctgcgccca gacctgctgc gtcgatggcg ctgactactc ttcgacctat     300
   ggtatcacca ccagcggtga ctccctgaac ctcaagttcg tcaccaagca ccagtacggc     360
   accaacgtcg gctcccgtgt ctatctgatg gagaacgaca ccaagtacca gatgttcgag     420
   ctcctcggca acgagttcac cttcgatgtc gatgtctcca acctgggctg cggtctcaac     480
   ggcgccctct acttcgtttc catggatgct gatggtggca tgagcaaata ctctggcaac     540
   aaggctggcg ccaagtacgg taccggctac tgcgatgctc agtgcccgcg cgacctcaag     600
   ttcatcaacg gcgaggccaa cgttgggaac tggaccccct cgaccaacga tgccaacgcc     660
   ggcttcggcc gctatggcag ctgctgctct gagatggatg tctgggaggc caacaacatg     720
   gctactgcct tcactcctca cccttgcacc accgttggcc agagccgctg cgaggccgac     780
   acctgcggtg gcacctacag ctctgaccgc tatgctggtg tttgcgaccc tgatggctgc     840
   gacttcaacg cctaccgcca aggcgacaag accttctacg gcaagggcat gactgtcgac 900
   accaacaaga agatgaccgt cgtcacccag ttccacaaga actcggctgg cgtcctcagc 960
   gagatcaagc gcttctacgt ccaggacggc aagatcattg ccaacgctga gtccaagatc 1020
   cccggcaacc ccggaaactc cattacccag gagtattgcg atgcccagaa ggtcgccttc 1080
   agtaacaccg atgacttcaa ccgcaagggc ggtatggctc agatgagcaa ggccctcgca 1140
   ggccccatgg tcctggtcat gtccgtctgg gatgaccact acgccaacat gctctggctc 1200
   gactcgacct accccatcga ccaggccggc gcccccggcg ccgagcgcgg tgcttgcccg 1260
   accacctccg gtgtccctgc cgagatcgag gcccaggtcc ccaacagcaa cgtcatcttc 1320
   tccaacatcc gtttcggccc catcggctcg accgtccctg gccttgacgg cagcaacccc 1380
   ggcaacccga ccaccaccgt cgttcctccc gcttctacct ccacctcccg tccgaccagc 1440
   agcactagct ctcccgtttc gaccccgact ggccagcccg gcggctgcac cacccagaag 1500
   tggggccagt gcggcggtat cggctacacc ggctgcacta actgcgttgc tggcaccacc 1560
   tgcactcagc tcaacccctg gtacagccag gtatgtttct cttccccctt ctagactcgc 1620
   ttggatttga cagttgctaa catctgctca acagtgcctg taa 1663
<210> 30
   <211> 39
   <212> DNA
   <213> Trichoderma reesei
<400> 30
   ttggatccga gtcgcagcgg caaccctagc ggcggcaac 39
<210> 31
   <211> 30
   <212> DNA
   <213> Trichoderma reesei
<400> 31
   taattctgca gttacaggca ctgagagtag 30
<210> 32
   <211> 41
   <212> DNA
   <213> Acremonium thermophilum
<400> 32
   ttggatccga gtcgcagcgg cgggaaccca ccccccgtca c 41
<210> 33
   <211> 34
   <212> DNA
   <213> Acremonium thermophilum
<400> 33
   taattctgca gtcacaggca ctgagagtac cagt 34
<210> 34
   <211> 28
   <212> DNA
   <213> Chaetomium thermophilum
<400> 34
   taatttacgt acctggcctt gacggcag 28
<210> 35
   <211> 30
   <212> DNA
   <213> Chaetomium thermophilum
<400> 35
   attaactgca gttacaggca ctgttgagca 30
<210> 36
   <211> 1096
   <212> DNA
   <213> Acremonium thermophilum
<220>
   <221> gene
   <222> (13)..(1091)
<220>
   <221> Intron
   <222> (84)..(142)
<220>
   <221> Intron
   <222> (392)..(514)
<400> 36
   ggactgcgca tcatgtatcg gaagttggcc gtcatctcgg ccttcttggc cacagctcgt     60
   gccctcgacg gaaagtcgac gaggtatgcc aatcctcgta cctctgccct ctgtagaaac    120
   aagtgaccga ctgcaaagac agatactggg actgctgcaa gccgtcctgc ggctgggccg    180
   gaaaggcctc ggtgaaccag cccgtcttct cgtgctcggc cgactggcag cgcatcagcg    240
   acttcaacgc gaagtcgggc tgcgacggag gctccgccta ctcgtgcgcc gaccagacgc    300
   cctgggcggt caacgacaac ttctcgtacg gcttcgcagc cacggccatc gccggcggct    360
   ccgagtccag ctggtgctgc gcctgctatg cgtgagttct ctgcaagccg cttcccaccc    420
   ccgctttctg tgcaggccgc ttccccccta cccacccact tccccccccc cgcctctgtg    480
   atcgggcatc cgagctaagt tgcgtgtcgt ccagactcac cttcaactcg ggccccgtcg    540
   cgggcaagac catggtggtg cagtcgacca gcaccggcgg cgacctgggc agcaaccagt    600
   tcgacctcgc catccccggc ggcggcgtgg gcatcttcaa cggctgcgcc tcccagttcg    660
   gcggcctccc cggcgcccag tacggcggca tcagcgaccg cagccagtgc tcgtccttcc    720
   ccgcgccgct ccagccgggc tgccagtggc gcttcgactg gttccagaac gccgacaacc    780
   ccaccttcac cttccagcgc gtgcagtgcc cgtccgagct cacgtcccgc acgggctgta    840
   agcgcgacga cgacgccagc tatcccgtct tcaacccgcc ttcgggcaac cctagcggcg    900
   gcaaccctcc cggcggaaac ccgcctggca ccaccaccac ccgccgccca gccactacca    960
   ctggaagctc tcccggacct acccagtctc actacggcca gtgcggcggt attggctaca   1020
   gcggccccac ggtctgcgcc agcggcacaa cttgccaggt cctgaaccct tactactctc   1080
   agtgcctgta actgca                                                   1096
<210> 37
   <211> 1102
   <212> DNA
   <213> Acremonium thermophilum
<220>
   <221> gene
   <222> (13)..(1097)
<220>
   <221> Intron
   <222> (84)..(142)
<220>
   <221> Intron
   <222> (392)..(514)
<400> 37
   ggactgcgca tcatgtatcg gaagttggcc gtcatctcgg ccttcttggc cacagctcgt      60
   gccctcgacg gaaagtcgac gaggtatgcc aatcctcgta cctctgccct ctgtagaaac     120
   aagtgaccga ctgcaaagac agatactggg actgctgcaa gccgtcctgc ggctgggccg     180
   gaaaggcctc ggtgaaccag cccgtcttct cgtgctcggc cgactggcag cgcatcagcg     240
   acttcaacgc gaagtcgggc tgcgacggag gctccgccta ctcgtgcgcc gaccagacgc     300
   cctgggcggt caacgacaac ttctcgtacg gcttcgcagc cacggccatc gccggcggct     360
   ccgagtccag ctggtgctgc gcctgctatg cgtgagttct ctgcaagccg cttcccaccc     420
   ccgctttctg tgcaggccgc ttccccccta cccacccact tccccccccc cgcctctgtg     480
   atcgggcatc cgagctaagt tgcgtgtcgt ccagactcac cttcaactcg ggccccgtcg     540
   cgggcaagac catggtggtg cagtcgacca gcaccggcgg cgacctgggc agcaaccagt     600
   tcgacctcgc catccccggc ggcggcgtgg gcatcttcaa cggctgcgcc tcccagttcg     660
   gcggcctccc cggcgcccag tacggcggca tcagcgaccg cagccagtgc tcgtccttcc     720
   ccgcgccgct ccagccgggc tgccagtggc gcttcgactg gttccagaac gccgacaacc     780
   ccaccttcac cttccagcgc gtgcagtgcc cgtccgagct cacgtcccgc acgggctgta     840
   agcgcgacga cgacgccagc tatcccgtct tcaacccgcc ttcgggcggg aacccacccc     900
   ccgtcaccac cacgaccacg accacgacca cgtcgaagcc gtcgcagccc accaccacga     960
   ccacgaccac cagcccgcag ggtccgcagc agacgcactg gggccagtgc ggcgggatcg    1020
   gctggacggg gccgcagtcg tgccagagcc cgtggacgtg ccagaagcag aacgactggt    1080
   actctcagtg cctgtgactg ca                                             1102
<210> 38
   <211> 1199
   <212> DNA
   <213> Acremonium thermophilum
<220>
   <221> gene
   <222> (13)..(1194)
<220>
   <221> Intron
   <222> (84)..(142)
<220>
   <221> Intron
   <222> (392)..(514)
<220>
   <221> Intron
   <222> (1122)..(1185)
<400> 38
   ggactgcgca tcatgtatcg gaagttggcc gtcatctcgg ccttcttggc cacagctcgt      60
   gccctcgacg gaaagtcgac gaggtatgcc aatcctcgta cctctgccct ctgtagaaac     120
   aagtgaccga ctgcaaagac agatactggg actgctgcaa gccgtcctgc ggctgggccg     180
   gaaaggcctc ggtgaaccag cccgtcttct cgtgctcggc cgactggcag cgcatcagcg     240
   acttcaacgc gaagtcgggc tgcgacggag gctccgccta ctcgtgcgcc gaccagacgc     300
   cctgggcggt caacgacaac ttctcgtacg gcttcgcagc cacggccatc gccggcggct     360
   ccgagtccag ctggtgctgc gcctgctatg cgtgagttct ctgcaagccg cttcccaccc     420
   ccgctttctg tgcaggccgc ttccccccta cccacccact tccccccccc cgcctctgtg     480
   atcgggcatc cgagctaagt tgcgtgtcgt ccagactcac cttcaactcg ggccccgtcg     540
   cgggcaagac catggtggtg cagtcgacca gcaccggcgg cgacctgggc agcaaccagt     600
   tcgacctcgc catccccggc ggcggcgtgg gcatcttcaa cggctgcgcc tcccagttcg     660
   gcggcctccc cggcgcccag tacggcggca tcagcgaccg cagccagtgc tcgtccttcc     720
   ccgcgccgct ccagccgggc tgccagtggc gcttcgactg gttccagaac gccgacaacc     780
   ccaccttcac cttccagcgc gtgcagtgcc cgtccgagct cacgtcccgc acgggctgta     840
   agcgcgacga cgacgccagc tatcccgtct tcaacccgcc ttcggtacct ggccttgacg     900
   gcagcaaccc cggcaacccg accaccaccg tcgttcctcc cgcttctacc tccacctccc     960
   gtccgaccag cagcactagc tctcccgttt cgaccccgac tggccagccc ggcggctgca    1020
   ccacccagaa gtggggccag tgcggcggta tcggctacac cggctgcact aactgcgttg    1080
   ctggcaccac ctgcactcag ctcaacccct ggtacagcca ggtatgtttc tcttccccct   1140
   tctagactcg cttggatttg acagttgcta acatctgctc aacagtgcct gtaactgca     1199
<210> 39
   <211> 298
   <212> PRT
   <213> Acremonium thermophilum
<400> 39
   Met Tyr Arg Lys Leu Ala Val Ile Ser Ala Phe Leu Ala Thr Ala Arg
   1               5                   10                  15
   Ala Leu Asp Gly Lys Ser Thr Arg Tyr Trp Asp Cys Cys Lys Pro Ser
               20                  25                  30
   Cys Gly Trp Ala Gly Lys Ala Ser Val Asn Gln Pro Val Phe Ser Cys
           35                  40                  45
   Ser Ala Asp Trp Gln Arg Ile Ser Asp Phe Asn Ala Lys Ser Gly Cys
       50                  55                  60
   Asp Gly Gly Ser Ala Tyr Ser Cys Ala Asp Gln Thr Pro Trp Ala Val
   65                  70                  75                  80
   Asn Asp Asn Phe Ser Tyr Gly Phe Ala Ala Thr Ala Ile Ala Gly Gly
                   85                  90                  95
   Ser Glu Ser Ser Trp Cys Cys Ala Cys Tyr Ala Leu Thr Phe Asn Ser
               100                 105                 110
   Gly Pro Val Ala Gly Lys Thr Met Val Val Gln Ser Thr Ser Thr Gly
           115                 120                 125
   Gly Asp Leu Gly Ser Asn Gln Phe Asp Leu Ala Ile Pro Gly Gly Gly
       130                 135                 140
   Val Gly Ile Phe Asn Gly Cys Ala Ser Gln Phe Gly Gly Leu Pro Gly
   145                 150                 155                 160
   Ala Gln Tyr Gly Gly Ile Ser Asp Arg Ser Gln Cys Ser Ser Phe Pro
                   165                 170                 175
   Ala Pro Leu Gln Pro Gly Cys Gln Trp Arg Phe Asp Trp Phe Gln Asn
               180                 185                 190
   Ala Asp Asn Pro Thr Phe Thr Phe Gln Arg Val Gln Cys Pro Ser Glu
           195                 200                 205
   Leu Thr Ser Arg Thr Gly Cys Lys Arg Asp Asp Asp Ala Ser Tyr Pro
       210                 215                 220
   Val Phe Asn Pro Pro Ser Gly Asn Pro Ser Gly Gly Asn Pro Pro Gly
   225                 230                 235                 240
   Gly Asn Pro Pro Gly Thr Thr Thr Thr Arg Arg Pro Ala Thr Thr Thr
                   245                 250                 255
   Gly Ser Ser Pro Gly Pro Thr Gln Ser His Tyr Gly Gln Cys Gly Gly
               260                 265                 270
   Ile Gly Tyr Ser Gly Pro Thr Val Cys Ala Ser Gly Thr Thr Cys Gln
           275                 280                 285
   Val Leu Asn Pro Tyr Tyr Ser Gln Cys Leu
       290                 295
<210> 40
   <211> 300
   <212> PRT
   <213> Acremonium thermophilum
<400> 40
   Met Tyr Arg Lys Leu Ala Val Ile Ser Ala Phe Leu Ala Thr Ala Arg
   1               5                   10                  15
   Ala Leu Asp Gly Lys Ser Thr Arg Tyr Trp Asp Cys Cys Lys Pro Ser
               20                  25                  30
   Cys Gly Trp Ala Gly Lys Ala Ser Val Asn Gln Pro Val Phe Ser Cys
           35                  40                  45
   Ser Ala Asp Trp Gln Arg Ile Ser Asp Phe Asn Ala Lys Ser Gly Cys
   50 55 60
   Asp Gly Gly Ser Ala Tyr Ser Cys Ala Asp Gln Thr Pro Trp Ala Val
   65 70 75 80
   Asn Asp Asn Phe Ser Tyr Gly Phe Ala Ala Thr Ala Ile Ala Gly Gly
   85 90 95
   Ser Glu Ser Ser Trp Cys Cys Ala Cys Tyr Ala Leu Thr Phe Asn Ser
   100 105 110
   Gly Pro Val Ala Gly Lys Thr Met Val Val Gln Ser Thr Ser Thr Gly
   115 120 125
   Gly Asp Leu Gly Ser Asn Gln Phe Asp Leu Ala Ile Pro Gly Gly Gly
   130 135 140
   Val Gly Ile Phe Asn Gly Cys Ala Ser Gln Phe Gly Gly Leu Pro Gly
   145 150 155 160
   Ala Gln Tyr Gly Gly Ile Ser Asp Arg Ser Gln Cys Ser Ser Phe Pro
   165 170 175
   Ala Pro Leu Gln Pro Gly Cys Gln Trp Arg Phe Asp Trp Phe Gln Asn
   180 185 190
   Ala Asp Asn Pro Thr Phe Thr Phe Gln Arg Val Gln Cys Pro Ser Glu
   195 200 205
   Leu Thr Ser Arg Thr Gly Cys Lys Arg Asp Asp Asp Ala Ser Tyr Pro
   210 215 220
   Val Phe Asn Pro Pro Ser Gly Gly Asn Pro Pro Pro Val Thr Thr Thr
   225 230 235 240
   Thr Thr Thr Thr Thr Thr Ser Lys Pro Ser Gln Pro Thr Thr Thr Thr
   245 250 255
   Thr Thr Thr Ser Pro Gln Gly Pro Gln Gln Thr His Trp Gly Gln Cys
   260 265 270
   Gly Gly Ile Gly Trp Thr Gly Pro Gln Ser Cys Gln Ser Pro Trp Thr
   275 280 285
   Cys Gln Lys Gln Asn Asp Trp Tyr Ser Gln Cys Leu
   290 295 300
<210> 41
   <211> 311
   <212> PRT
   <213> Acremonium thermophilum
<400> 41
   Met Tyr Arg Lys Leu Ala Val Ile Ser Ala Phe Leu Ala Thr Ala Arg
   1               5                   10                  15
   Ala Leu Asp Gly Lys Ser Thr Arg Tyr Trp Asp Cys Cys Lys Pro Ser
               20                  25                  30
   Cys Gly Trp Ala Gly Lys Ala Ser Val Asn Gln Pro Val Phe Ser Cys
           35                  40                  45
   Ser Ala Asp Trp Gln Arg Ile Ser Asp Phe Asn Ala Lys Ser Gly Cys
       50                  55                  60
   Asp Gly Gly Ser Ala Tyr Ser Cys Ala Asp Gln Thr Pro Trp Ala Val
   65                  70                  75                  80
   Asn Asp Asn Phe Ser Tyr Gly Phe Ala Ala Thr Ala Ile Ala Gly Gly
                   85                  90                  95
   Ser Glu Ser Ser Trp Cys Cys Ala Cys Tyr Ala Leu Thr Phe Asn Ser
               100                 105                 110
   Gly Pro Val Ala Gly Lys Thr Met Val Val Gln Ser Thr Ser Thr Gly
           115                 120                 125
   Gly Asp Leu Gly Ser Asn Gln Phe Asp Leu Ala Ile Pro Gly Gly Gly
       130                 135                 140
   Val Gly Ile Phe Asn Gly Cys Ala Ser Gln Phe Gly Gly Leu Pro Gly
   145                 150                 155                 160
   Ala Gln Tyr Gly Gly Ile Ser Asp Arg Ser Gln Cys Ser Ser Phe Pro
                   165                 170                 175
   Ala Pro Leu Gln Pro Gly Cys Gln Trp Arg Phe Asp Trp Phe Gln Asn
               180                 185                 190
   Ala Asp Asn Pro Thr Phe Thr Phe Gln Arg Val Gln Cys Pro Ser Glu
           195                 200                 205
   Leu Thr Ser Arg Thr Gly Cys Lys Arg Asp Asp Asp Ala Ser Tyr Pro
       210                 215                 220
   Val Phe Asn Pro Pro Ser Val Pro Gly Leu Asp Gly Ser Asn Pro Gly
   225                 230                 235                 240
   Asn Pro Thr Thr Thr Val Val Pro Pro Ala Ser Thr Ser Thr Ser Arg
                   245                 250                 255
   Pro Thr Ser Ser Thr Ser Ser Pro Val Ser Thr Pro Thr Gly Gln Pro
               260                 265                 270
   Gly Gly Cys Thr Thr Gln Lys Trp Gly Gln Cys Gly Gly Ile Gly Tyr
           275                 280                 285
   Thr Gly Cys Thr Asn Cys Val Ala Gly Thr Thr Cys Thr Gln Leu Asn
       290                 295                 300
   Pro Trp Tyr Ser Gln Cys Leu
   305                 310
<210> 42
   <211> 20
   <212> DNA
   <213> Acremonium thermophilum
<400> 42
   gcagcaacca gttcgacctc 20
<210> 43
   <211> 36
   <212> DNA
   <213> Acremonium thermophilum
<400> 43
   ttaactgcag tcactgggca gtgcagccac cgcctc 36
<210> 44
   <211> 20
   <212> DNA
   <213> Acremonium thermophilum
<400> 44
   actgctgcaa gccgtcctgc 20
<210> 45
   <211> 41
   <212> DNA
   <213> Acremonium thermophilum
<400> 45
   ttaactgcag tcaaccgcta ggcgggttga agacgggata g 41

## Description

### Field of the Invention

The present invention relates to novel cellulase enzymes, especially novel endoglucanases including endoglucanase fusion proteins, preparations and compositions containing these endoglucanase enzymes and fusion proteins, expression vectors, host cells and methods for their preparation and uses of the cellulases, preparations and compositions in the textile, detergent, and pulp and paper industries.

### Background of the Invention

Cellulose is the major structural component of higher plants and occurs naturally in almost pure form only in cotton fiber. It provides plant cells with high tensile strength helping them to resist mechanical stress and osmotic pressure. Cellulose is a linear polysaccharide of glucose residues connected by β-1,4 linkages. In nature, cellulose is usually associated with lignin together with hemicelluloses, such as xylans and glucomannans. Cellulolytic enzymes hydrolyze cellulose and are produced by a wide variety of bacteria and fungi. Cellulases are industrially important enzymes with a current annual market value of about 190 million US $. In the textile industry, cellulases are used in denim finishing to create a fashionable stone washed appearance in denim cloths in a biostoning process, and they are also used, for instance, to clean fuzz and prevent formation of pills on the surface of cotton garments. In detergent industry cellulases are used to brighten colors and to prevent graying and pilling of garments. Cellulases are further used in food industry and animal feed manufacturing, and they have a great potential in the pulp and paper industry, for instance, in deinking to release ink from fiber surfaces and in improving pulp drainage. The wide spectrum of industrial uses for cellulases has established a need for commercial cellulase products containing different cellulase components and functioning optimally in different pH and temperature ranges.

The practical use of cellulases is hampered by the nature of the known cellulase compositions, which are often enzyme mixtures having a variety of activities and substrate specificities. For this reason, efforts have been made to obtain cellulases having only the desired activities. The unique properties of each cellulase make some more suitable for certain purposes than others. While the enzymes differ in a number of ways, one of the most important differences is the pH optimum. Neutral cellulases are most active in the pH range 6-8 and alkaline cellulases in the pH range 7.5-10, whereas acid cellulases, having the pH optimum at pH 4.5-5.5, show very low activity levels at higher pH values. Neutral and acid cellulases are especially useful in the textile industry. In fabric treatment cellulases attack the chains of cellulose molecules that form the cotton fibers, thereby affecting the characteristics of the fabric.

In textile industry "stone washed" look or an abraded look has been denim producers' interest in recent years. Traditional stone washing with pumice stones reduces the strength of fabric and burdens the laundering apparatuses. The trend has been towards enzymatic denim finishing processes and cellulases have replaced or are being used together with pumice stones to give the fabric its desired "worn" look. Controlled enzyme treatments result in less damage to the garments and machines and eliminate the need for disposal of stones.

Additionally, textile industry uses cellulases in biofinishing, i.e. to create permanent improvement of depilling and improved pilling resistance, cleared surface structure by reduced fuzz, improved textile handle, such as softness, smoothness and a silkier feel, improved drapability and brighter colors of the textile and improved moisture absorbability.

Cellulases applied in denim treatment are usually divided into two main groups: acid and neutral cellulases. Acid cellulases typically operate at pH 4.0-5.5 and the neutral cellulases in the range of pH 6-8. Acid cellulases used in biostoning mainly originate from *Trichoderma reesei* (sexual form *Hypocrea jecorina*) and the neutral cellulases come from a variety of fungi, including genera of *Melanocarpus, Humicola, Myceliophthora, Fusarium, Acremonium,* and *Chrysosporium* (Haakana *et al.* 2004). *T. reesei* enzymes include, e.g., cellulases from the glycoside family 5 (endoglucanase II, EGII), family 7 (cellobiohydrolase I, CBHI) and family 12 (endoglucanase III, EGIII; Ward *et al.* 1993), and the neutral cellulases, most often endoglucanases, from family 45 and family 7 (Henrissat, 1991; Henrissat and Bairoch, 1993, 1996).

Cellulases comprise a catalytic domain/core (CD) expressing cellulase activity. In addition to the catalytic domain the cellulase molecule may comprise one or more cellulose binding domains (CBDs), also named as carbohydrate binding domains/modules (CBD/CBM), which can be located either at the N- or C-terminus of the catalytic domain. CBDs have carbohydrate-binding activity and they mediate the binding of the cellulase to crystalline cellulose but have little or no effect on cellulase hydrolytic activity of the enzyme on soluble substrates. These two domains are typically connected via a flexible and highly glycosylated linker region.

Cellulases that attack primarily on the surface of the fiber are especially useful in stone washing of denim dyed with Indigo dye, as the dye is located on the surface of the fiber. When used to treat cotton fabric, acid cellulases generally require a shorter washing time than neutral cellulases. Acid cellulases are especially used in biofinishing (depilling) and also in denim treatment (biostoning).

Endoglucanases (EGs) in connection of the present invention mean enzymes classified as E.C. 3.2.1.4 and are one of the three types of cellulases generally needed for the biological conversion of cellulose to glucose. Endoglucanases cut internal beta-1,4-glucosidic bonds, whereas cellobiohydrolases cut the disaccharide cellobiose from the end of the cellulose polymer chain and beta-1,4-glucosidases hydrolyze the cellobiose and other short cello-oligosaccharides to glucose. Some naturally occurring endoglucanases have a cellulose-binding domain (CBD), while others do not.

Also endoglucanases are widely used in textile, detergent, and pulp and paper industry. For instance, endoglucanases of the cel45 family (EGs fam 45) are described e.g. in U.S. Patent No. 6,001,639, which describes enzymes having endoglucanase activity and having two conserved amino acid sequences. Uses in textile, detergent, and pulp and paper applications are generally discussed and treating of lignocellulosic material is mentioned. WO 2004/053039 is directed to detergent applications of endoglucanases. U.S. Patent No. 5,958,082 discloses the use of endoglucanase, especially from *Thielavia terrestris* in textile applications providing stoned washed or abraded look of twill jeans. EP 0495258 relates to detergent compositions containing *Humicola* cellulase. U.S. Patent No. 5,948,672 describes a cellulase preparation containing endoglucanase, especially from *Humicola* and its use in textile and pulp applications. WO98/12307 provides endoglucanase variants from e.g. *Thielavia terrestris* and *Myceliophora themophila* for use in textile applications. Also a recombinant endoglucanase of *Acremonium* is disclosed. It does not show high identity to that of the present invention.

EG:s and EG-enriched compositions and concentrates are also commercially available.

However, there is a continuous need for improved cellulases, including endoglucanases that are more efficient in fabric treatment and in other fields, where cellulases traditionally are used. In particular, there is a continuous need for more efficient cellulases to improve the process economics.

The present invention aims to meet this need.

### Brief description of the invention

An object of the present invention is to provide novel endoglucanases and endoglucanase fusion proteins having improved hydrolytic properties for use in textile industry, especially in cotton finishing processes, such as in depilling, and stone washing denim, and for use in detergent compositions as well as in other fields. The novel endoglucanases and endoglucanase fusion proteins of the invention have the advantage of being active at acid and neutral pH values, they have highly improved performance in textile biofinishing and biostoning applications and in detergent applications. When used in treating cellulose-containing textile materials, the novel endoglucanases and endoglucanase fusion proteins provide a smooth feel, improved appearance and softness as well as permanent depilling to the textile. With the improved efficiency of the endoglucanases of the invention, the use of the enzymes is significantly more economical. Additional advantages are achieved also in terms of logistics and the storage of the enzyme products, when smaller amounts of the enzyme product are needed. Furthermore, the novel endoglucanases and endoglucanase fusion proteins of the present invention, as being acidic, act more rapidly, affording time- and cost-effective treatment procedures and savings in equipment as well as treatment facilities.

A further object of the present invention is to provide polynucleotides encoding the novel endoglucanases and endoglucanase fusion proteins of the present invention.

A still further object of the present invention is to provide novel expression plasmids or vectors containing such polynucleotides, useful for the production of the novel endoglucanases and endoglucanase fusion proteins of the present invention, as well as novel hosts transformed with said expression plasmids.

A still further object of the present invention is to provide enzyme preparations, which contain one or more novel endoglucanases and endoglucanase fusion proteins having improved hydrolytic properties.

A still further object of the present invention is to provide methods of using the enzyme preparations and the endoglucanases and endoglucanase fusion proteins for finishing of textiles, especially for biofinishing and biostoning of denim.

A still further object of the present invention is to provide means for the use of the enzyme preparations of the invention in detergent compositions.

The present invention relates to an endoglucanase polypeptide comprising cellulolytic activity and being selected from the group consisting of:
a) a polypeptide comprising an amino acid sequence having at least 95% sequence identity to SEQ ID NO: 2;
b)a polypeptide of a) lacking the carbohydrate binding domain, or the carbohydrate binding domain and the linker region..

The present invention further relates to an endoglucanase fusion protein comprising a cellulolytically active fragment of a polypeptide having an amino acid sequence having at least 78 % sequence identity to SEQ ID NO 2, attached to a heterologous carbohydrate binding domain (CBD).

The present invention also relates to an endoglucanase fusion protein comprising an amino acid sequence derived from a polypeptide comprising an amino acid sequence having at least 95% sequence identity to SEQ ID NO: 2 attached to a carbohydrate binding domain. The fusion protein may additionally comprise a linker region.

The present invention further relates to an isolated polynucleotide encoding the above defined endoglucanase polypeptide or fusion protein. Especially the invention relates to a polypeptide selected from the group consisting of:
a) a nucleotide sequence of SEQ ID NO: 1, SEQ ID NO: 16, SEQ ID NO: 18, SEQ ID NO: 36, SEQ ID NO: 37, or SEQ ID NO: 38;
b) a complementary strand of a);
   and
c) a sequence that is degenerate as a result of the genetic code to any one of the sequences as defined in a), or b).

The present invention further relates to an expression vector comprising the above defined polynucleotide sequence.

The present invention further relates to novel hosts transformed with the vectors of the invention, especially hosts that are capable of high level expression of the endoglucanase or endoglucanase fusion protein of the invention.

The present invention further relates to an enzyme preparation, which contains one or more endoglucanases or endoglucanase fusion proteins of the invention.

The present invention further relates to methods for using the enzyme preparations of the invention for the biofinishing of textiles, especially for depilling.

The present invention further relates to methods for using the enzyme preparations of the invention for the finishing of textiles, especially for biostoning of denim.

The present invention further relates to the use of the enzyme preparations of the invention in detergent compositions.

The present invention still further relates to an *Escherichia coli* strain having accession number DSM 17324, DSM 18813, DSM 18814, DSM 18815, DSM 18816 or DSM 18817.

### Brief description of the drawings

Figure 1 illustrates the schematic picture of the expression cassettes used in the transformation of *Trichoderma reesei* protoplasts for production of *Acremonium thermophilum* cellulases of the invention. The recombinant genes were under control of *T. reesei cbh*I/*cel7A* promoter (cbhl prom) and transcription termination was ensured with the addition of the *T. reesei cbh*I terminator (cbhl term). The *amd*S gene (*amd*S) was included for selection of the transformants.
Figure 2 illustrates the pH dependency of the heterologously produced *A. thermophilum* EG_40 and EG_40_like cellulases by determining from the culture supernatant using CMC as substrate in a 10 min reaction at 50°C (A). The temperature optimum of EG_40 and EG_40_like cellulases was determined at pH 5.5 and 5, respectively. The reaction with CMC as a substrate was performed for 60 min. BSA (100 µg/ml) was added as a stabilizer. (B).
Figure 3 shows the performance of EG_40 celluase in biostoning at different temperatures evaluated by measuring the color.
Figure 4 shows the biostoning effect of a mixture of EGII enriched and EG_40 concentrates as compared to a prior art EGII-enriched concentrate.
Figure 5 shows series of photographs showing the depilling effect of an endoglucanase of the invention.

### Detailed description of the invention

The present invention is based on efforts to find further improved cellulases for use in textile industry. Surprisingly it was found that, starting from an *Acremonium species,* novel endoglucanases could be isolated and recombinant enzymes could be produced, which endoglucanases not only have an acceptable temperature profile but also show unexpected favorable depilling performance and are at least four times as efficient as a commercial EG-containing preparation. Additionally, the novel endoglucanases showed excellent biostoning properties as compared to prior art cellulases.

Accordingly, the present invention relates to an endoglucanase polypeptide comprising a fragment having cellulolytic activity and being selected from the group consisting of:
a) a polypeptide comprising an amino acid sequence having at least 95% sequence identity to SEQ ID NO: 2, and
b) a polypeptide of a) or b)lacking the carbohydrate binding domain, or the carbohydrate binding domain and the linker region.

In one preferred embodiment of the invention said amino acid has at least 98 % sequence identity to SEQ ID NO: 2.

In yet another preferred embodiment of the invention said amino acid has SEQ ID NO: 2.

In yet another preferred embodiment of the invention said fragment has the amino acid sequence of SEQ ID NO: 17 or SEQ ID NO: 19.

In yet another preferred embodiment of the invention the polypeptides are obtainable or originate from an *Acremonium sp.,* preferably from *Acremonium thermophilum.*

The endoglucanase polypeptides of the present invention are preferably recombinant polypeptides. The recombinant polypeptides may be produced in a heterologous host or in a genetically modified homologous host.

According to one particular embodiment of the invention the endoglucanase polypeptide is a modification of a polypeptide having at least 95% sequence identity to SEQ ID NO: 2, wherein the carbohydrate binding domain CBD has been replaced by a heterologous CBD. In addition the linker region and/or signal sequence may be replaced by a heterologous linker region and/or signal sequence. These modified endoglucanases are also encompassed herein by the term endoglucanase fusion protein. In one preferred embodiment of the invention, the modified/fused endoglucanase has SEQ ID NO: 39, SEQ ID NO: 40 or SEQ ID NO: 41.

In one particular embodiment of the invention the fusion protein is an endoglucanase fusion protein comprising a cellulolytically active fragment of a polypeptide having an amino acid sequence having at least 95 % sequence identity to SEQ ID NO 2, and a heterologous carbohydrate binding domain (CBD), wherein the heterlogous CBD and the optional linker region are derived from *Trichoderma reesei* CBHI, *Chaetomium thermophilum* CBHI, or *Acremonium* sp. xylanase. "Heterologous" as used in the present context means that the CBD and possible linker part of the endoglucanase fusion protein are obtained from another organism and attached to the cellulolytically active core of the endoglucanase by gene modification. Especially the fusion protein comprises an amino acid sequence that has at least 71% sequence identity to SEQ ID NO: 39, at least 69% sequence identity to SEQ ID NO: 40, or at least 69% sequence identity to SEQ ID NO: 41. Especially the fusion protein comprises an amino acid sequence having 75, 80, 85, 90, 95, or 98% sequence identity to SEQ ID NO: 39, SEQ ID NO: 40 or SEQ ID NO: 41.

The present invention further relates to an isolated polynucleotide encoding the above defined endoglucanase polypeptide.

Specifically in one embodiment of the invention the isolated polynucleotide has a nucleotide sequence selected from the group consisting of:
a) a nucleotide sequence of SEQ ID NO:1, SEQ ID NO: 16, SEQ ID NO: 18, SEQ ID NO: 36, SEQ ID NO: 37, or SEQ ID NO: 38;
b) a complementary strand of a); and
c) a sequence that is degenerate as a result of the genetic code to any one of the sequences as defined in a), or b).

The present invention further relates to an expression vector comprising the above defined polynucleotide sequence.

The present invention further relates to novel hosts transformed with the vectors of the invention, especially hosts that are capable of high level expression of the endoglucanase or endoglucanase fusion protein of the invention. According to a preferred embodiment of the invention the enzymes are obtainable from *Acremonium thermophilum* strain ALKO4245 deposited as CBS116240.

The present invention further relates to an enzyme preparation, which contains one or more endoglucanases or endoglucanase fusion proteins of the invention.

The present invention further relates to methods for using the enzyme preparations of the invention for the biofinishing of textiles, especially for depilling.

The present invention further relates to methods for using the enzyme preparations of the invention for the finishing of textiles, especially for biostoning of denim.

The present invention further relates to the use of the enzyme preparations of the invention in detergent compositions.

The endoglucanase and endoglucanase fusion protein preparations of the invention are especially useful in the textile and detergent industry. They are especially useful in the textile industry for biofinishing of fabrics or garments, e.g., depilling, defuzzing, color clarification, harshness reduction, creation of different finishes (for example, a 'peach skin,' 'worn out,' 'sand washed,' or 'antique look' effect) and for biofinishing of yarn, for example, reduction of hairiness and improvement of smoothness. Additional uses include the use in detergent compositions to improve fabric care properties by antipilling, antigraying, color clarification and softening, and to improve textile-cleaning effect, for instance soil removal. Additional uses further include the use in biostoning of denim.

In cotton fabric, fuzz (microfibres) emerges from the surface, which may entangle during processing, thus forming pills. Enzymes weaken the microfibres raising up from the surface and shear forces of the treatment then remove them (Nierstrasz and Warmoeskerken, 2003). As used in the present context the expression "biofinishing" (also called depilling, defuzzing or bi-opolishing) refers to the use of enzymes in a controlled hydrolysis of cellulosic fibers in order to modify the fabric or yarn surface in a manner that prevents permanently pilling, improves fabric handle like softness and smoothness, clears the surface structure by reducing fuzzing, which results in clarification of colors, improves the drapability of the fabric, improves moisture absorbability, which may improve also the dyeability. Cellulase enzymes are used for treating or finishing of cellulose-containing textile materials, such as cotton, flax, ramie, jute, viscose, modal, lyocell and cupro, or blends thereof.

As used in the present context the expression "biostoning" of fabric or garment means the use of enzymes in place of, or in addition to, pumice stones for the treatment of fabric or garment, especially denim.

As used in the present context the expression "backstaining" refers to the tendency of released dye to redeposit on the surface of the fabric fibers.

As used in the present context the expression "detergent" refers to a cleansing agent that can contain surface active agents (anionic, non-ionic, cationic and ampholytic surfactants), builders and other optional incredients such as anti-redeposition and soil suspension agents, optical brighteners, bleaching agents, dyes and pigments and hydrolases. Suitable listing of the contents of detergents is given in U.S. Patent No. 5,433,750, a suitable list of surfactants is given in U.S. Patent No. 3,664,961.

The biological activity of an endoglucanase is its catalytic activity, and/or its ability to bind to cellulosic material. Cellulolytic activity of an endoglucanase is its hydrolytic activity.

As used in the present context the expression *"Acremonium sp."* refers especially to a filamentous fungal genus having the characteristics of the strain CBS 116240. This strain is presently classified as *A. thermophilum.*

As used in the present context "polynucleotide" refers to both RNA and DNA, and it may be single stranded or double stranded. The polynucleotide may also be a fragment of said polynucleotides comprising at least 20 nucleotides, e.g. at least 25, 30 or 40 nucleotides. According to one embodiment of the invention it is at least 100, 200 or 300 nucleotides in length. Further the polynucleotide may be degenerate as a result of the genetic code to any one of the sequences as defined above. This means that different codons may code for the same amino acid.

The novel polypeptides may also be variants of said polypeptides. A "variant" may be a polypeptide that occurs naturally e.g. as an allelic variant within the same strain, species or genus, or it may have been generated by mutagenesis. It may comprise amino acid substitutions, deletions or insertions, but it still functions in a substantially similar manner to the enzymes defined above i.e. it comprises a fragment having cellulolytic activity.

An expression vector is a cloning plasmid or vector capable of expressing DNA encoding the endoglucanases and endoglucanase fusion proteins of the invention after transformation into a desired host. When a fungal host is used, the gene of interest is preferably provided to a fungal host as part of a cloning or expression vehicle that integrates into the fungal chromosome, or allows the gene of interest to integrate into the host chromosome, or as an autonomously replicating plasmid. Sequences that are part of the cloning vehicle or expression vehicle may also be integrated with said DNA during the integration process. In addition, in fungi the expression vector or parts thereof can be targeted into predetermined loci.

The DNA encoding the endoglucanases and the endoglucanase fusion proteins of the invention is also preferably placed under the control of (i.e., operably linked to) certain control sequences such as promoter sequences provided by the vector (which integrate with the gene of interest). Alternatively, the control sequences can be those at the insertion site.

The expression control sequences of an expression vector will vary depending on whether the vector is designed to express a certain gene in a prokaryotic or in a eukaryotic host (for example, a shuttle vector may provide a gene for selection in bacterial hosts). Expression control sequences can contain transcriptional regulatory elements such as promoters, enhancer elements, and transcriptional termination sequences, and/or translational regulatory elements, such as translational initiation and termination sites.

A polynucleotide molecule, such as DNA, is said to be "capable of expressing" a polypeptide if it contains expression control sequences which contain transcriptional regulatory information and such sequences are "operably linked" to the nucleotide sequence which encodes the polypeptide.

An operable linkage is a linkage in which a sequence is connected to a regulatory sequence (or sequences) in such a way as to place expression of the sequence under the influence or control of the regulatory sequence. Two DNA sequences (such as a promoter region sequence linked to the 5' end of the protein encoding sequence) are said to be operably linked if function of promoter results in the transcription.

The vectors of the invention may further comprise other operably linked regulatory elements, such as enhancer sequences.

In a preferred embodiment, genetically stable transformants are constructed whereby the DNA encoding the endoglucanases or endoglucanase fusion proteins of the invention is integrated into the host chromosome by transformation with a vector, which harbors sequences promoting integration of said vector into the chromosome.

Cells that have stably integrated DNA encoding the endoglucanases or the endoglucanase fusion proteins of the invention into their chromosomes are selected by also introducing one or more markers, homologous or heterologous, which allow for selection of host cells which contain the expression vector in the chromosome, for example the marker may provide biocide resistance, e.g., resistance to antibiotics, or heavy metals, such as copper, or markers complementing an auxotrophic mutation in the host chromosome, and the like. The selectable marker gene can either be directly linked to the DNA gene sequences to be expressed, or introduced into the same cell by co-transformation.

Once the vector or DNA sequence of the invention containing the construct(s) is prepared for expression, the DNA construct(s) is introduced into an appropriate host cell by any of a variety of suitable means, including transformation as known in the art. After the introduction of the vector, recipient cells are grown in a selective medium, which selects for the growth of transformed cells.

Suitable expression and production host systems are for example the production system developed for the fungus host *Trichoderma* (EP 244 234), or *Aspergillus* production system, such as *A. oryzae* or *A. niger* (WO 9708325 and WO 9533386, U.S. Patent No. 5,843,745, U.S. Patent No. 5,770,418), or the production system developed for *Fusarium,* such as *F. oxysporum* (Malardier *et al.,* 1989). Suitable production systems developed for bacteria are a production system developed for *Bacillus,* for example *B. subtilis* or for *E. coli,* or for actinomycete *Streptomyces.* Suitable production systems developed for yeasts are systems developed for *Saccharomyces, Shizosaccharomyces* or *Pichia pastoris.* Production systems in some other microbes or in mammalian cells or in plants are also possible.

Expression of the cloned gene sequence(s) results in the production of the desired protein, or in the production of a fragment of this protein. This expression can take place in a continuous manner in the transformed cells, or in a controlled manner.

Fragments are understood to be parts of polypeptide or nucleic acid molecules long enough to have the desired enzymatic properties or to code for the described endoglucanases or endoglucanase fusion proteins or a biologically active fragment thereof. The term "degenerate" means that the nucleotide sequence may vary as long as the encoded amino acid sequence is the same. This is so, because there is more than one nucleotide triplet that codes for a single amino acid.

As used in the present context the term sequence "identity" refers to the global identity between two amino acid sequences compared to each other from the first amino acid encoded by the corresponding gene to the last amino acid. The identity of the full-length sequences is measured by using Needleman-Wunsch global alignment program at EMBOSS (European Molecular Biology Open Software Suite; Rice *et al.,* 2000) program package, version 3.0.0, with the following parameters: EMBLOSUM62, Gap penalty 10.0, Extend penalty 0.5. The algorithm is described in Needleman and Wunsch (1970). The man skilled in the art is aware of the fact that results using Needleman-Wunsch algorithm are comparative only when aligning corresponding domains of the sequence. Consequently comparison of e.g. cellulase sequences including CBD or signal sequences with sequences lacking those elements cannot be done.

Cellulolytic enzymes useful for hydrolyzing cellulosic material are obtainable or originate from *Acremonium sp.,* preferably *A. thermophilum.* "Obtainable from" or "originating from" means that they can be obtained from said species, but it does not exclude the possibility of obtaining them from other sources. In other words they may originate from any organism including plants. Preferably they originate from microorganisms e.g. bacteria or fungi. The bacteria may be for example from a genus selected from *Bacillus, Azospirillum* and *Streptomyces.* More preferably the enzyme originates from fungi (including filamentous fungi and yeasts), for example from a genus selected from the group consisting of *Thermoascus, Acremonium, Chaetomium, Achaetomium, Aspergillus, Botrytis, Chrysosporium, Collybia, Fomes, Fusarium, Humicola, Hypocrea, Lentinus, Melanocarpus, Myceliophthora, Myriococcum, Neurospora, Penicillium, Phanerochaete, Phlebia, Pleurotus, Podospora, Polyporus, Rhizoctonia, Scytalidium, Pycnoporus, Trametes* and *Trichoderma.*

As used in the present context the expressions "enzyme preparation", "cellulase preparation" and "endoglucanase preparation" refer to any enzyme product, which contains at least one endoglucanase or endoglucanase fusion protein of the invention. Thus, such an enzyme preparation may be a spent culture medium or filtrate containing one or more endoglucanases or endoglucanase fusion proteins or one or more endoglucanases or endoglucanase fusion proteins and other enzymes, an isolated endoglucanase or endoglucanase fusion protein or a mixture of one or more endoglucanases or endoglucanase fusion proteins or a mixture of one or more endoglucanases or endoglucanase fusion proteins and one or more other enzymes. In addition to the endoglucanase activity, such a preparation may contain additives, such as stabilizers, buffers, preservatives, surfactants and/or culture medium components. Preferred additives are such, which are commonly used in enzyme preparations intended for the application, where the enzyme preparation is used. The enzyme preparation may be in the form of liquid, powder or granulate.

By "spent culture medium" is here meant the culture medium of the host comprising the produced enzymes. Preferably the host cells are separated from the said medium after the production.

The enzyme preparation may comprise one or more endoglucanases or endoglucanase fusion proteins of the present invention or other cellulase enzymes together with one or more endoglucanases or endoglucanase fusion proteins of the present invention. For example, endoglucanases having different properties may be combined to make the enzyme preparation more useful for different conditions.

To obtain the enzyme preparations of the invention, the hosts having the desired properties (that is, hosts capable of expressing economically feasible quantities of the endoglucanases or endoglucanase fusion proteins of the invention) are cultivated under suitable conditions, the desired enzymes are secreted from the hosts into the culture medium, and the enzyme preparation is recovered from said culture medium by methods known in the art.

The enzyme preparation may comprise, in addition to the endoglucanase or the endoglucanase fusion protein, one or more other enzymes, which may be for example amylases, lipases, proteases, pectinases and/or oxidases, such as laccases and peroxidases. Alternatively, before, during or after the treatment with the endoglucanase or the endoglucanase fusion protein of the present invention, another enzyme treatment may be carried out. The enzyme treatment may comprise, for example, one or more amylase treatments, one or more cellulase treatments and/or one or more peroxidase and/or lac-case treatments. Which other enzymes are included to the enzyme preparation or are used in the enzyme treatment, depends on the application.

The enzyme preparation can be the culture medium with or without the native or transformed host cells, or is recovered from the same by the application of methods well known in the art. However, because the endoglucanases or the endoglucanase fusion proteins of the invention are secreted into the culture media and display activity in the ambient conditions of the cellulolytic liquor, it is an advantage of the invention that the enzyme preparations of the invention may be utilized directly from the culture medium with no further purification. If desired, such preparations may be lyophilized or the enzymatic activity otherwise concentrated and/or stabilized for storage. The enzyme preparations of the invention are very economical to provide and use because (1) the enzymes may be used in a crude form; isolation of a specific enzyme from the culture medium is unnecessary and (2) because the enzymes are secreted into the culture medium, only the culture medium need be recovered to obtain the desired enzyme preparation; there is no need to extract an enzyme from the hosts. Preferably the host for such production is *Trichoderma,* and especially *T. reesei.*

The enzyme preparations of the invention may be provided as a liquid or as a solid, for example, in a dried powder or granular or liquid form, especially non-dusting granules, or a stabilized liquid, or the enzyme preparation may be otherwise concentrated or stabilized for storage or use. It is envisioned that enzyme preparations containing one or more of the cellulases of the invention can be further enriched or made partially or completely deficient in specific enzymatic activities, so as to satisfy the requirements of a specific utility in various applications e.g. in the textile industry. A mixture of enzyme activities secreted by a host and especially a fungal host, can be chosen to be advantageous in a particular industrial application, for example biofinishing and biostoning.

The enzyme preparations of the invention can be adjusted to satisfy the requirements of specific needs in various applications in the textile, detergent or the pulp and paper industry.

Blends may be prepared with other macromolecules that are not necessarily all produced from the same host (for example, other enzymes such as endoglucanases, amylases, lipases, proteases, pectinases and/or oxidases, such as laccases and peroxidases) or chemicals that may enhance the performance, stability, or buffering of the desired enzyme preparation. Non-dusting granules may be coated. Liquid enzyme preparations can be stabilized by adding a polyol such as propylene glycol, a sugar or sugar alcohol, lactic acid or boric acid, or sodium chloride, according to established methods.

Protected forms of the enzymes of the invention may be prepared as described in EP 238,216.

The enzyme preparations of the invention can contain a surfactant which can be anionic, non-ionic, cationic, amphoteric or a mixture of these types, especially when used as a detergent composition, Useful detergent compositions are described e.g. in WO 94/07998, U.S. Patent No. 5,443,750 and U.S. Patent No. 3,664,961.

If required, a desired enzyme may be isolated, and further purified in accordance with conventional conditions, such as extraction, precipitation, chromatography, affinity chromatography, electrophoresis, or the like. Isolated polypeptide in this context may simply mean that the cells and cell debris have been removed from the culture medium containing the polypeptide. Conveniently the polypeptides are isolated e.g. by adding anionic and/or cationic polymers to the spent culture medium to enhance precipitation of cells, cell debris and some enzymes that have unwanted side activities. The medium is then filtrated using an inorganic filtering agent and a filter to remove the precipitants formed. After this the filtrate is further processed using a semi-permeable membrane to remove excess of salts, sugars and metabolic products.

The enzyme preparations of this invention are especially useful in textile industry preferably in biofinishing and in biostoning or in detergent industry. Other useful areas are in pulp and paper industry.

"Biofinishing" refers to the use of enzymes in a controlled hydrolysis of cellulosic fibers in order to modify the fabric or yarn surface in a manner that prevents permanently pilling, improves fabric handle like softness and smoothness, clears the surface structure by reducing fuzzing, which results in clarification of colors, improves the drapability of the fabric, improves moisture absorbability and which may improve also the dyeability.

Enzymatic depilling can be carried out at any stage during textile wet processing, preferably after desizing and bleaching. The enzymatic process requires equipment with sufficient shear forces and mixing such as jet winch or washing machine (Nierstrasz V.A. and Warmoeskerken M.M.C.G., 2003).

Biofinishing is typically performed at about pH 4.0-6.0. The temperature of the reaction can range from about 30°C to 70°C, and is preferably 50-60°C. The liquor ratio (the ratio of the volume of liquid per weight of fabric) may range from about 3:1 to 20:1, preferably 5:1 to 10:1. The incubation time is generally 15 to 90 minutes, preferably 30 to 60 min. The enzyme dosage depends greatly on the type of the fabrics, machinery, process conditions (pH, temperature, liquor ratio, treatment time, denim load, process scale) and type of enzyme preparation and like. A person skilled in art is capable in defining suitable dosages and conditions.

The endoglucanases and endoglucanase fusion proteins of the invention are especially useful in the textile industry for biofinishing of fabrics or garments e.g. depilling, defuzzing, color clarification, harshness reduction, the creation of different finishes (for example, a 'peach skin,' 'wornout,' 'sand washed,' or 'antique look' effect) and biofinishing of yarn (for example reduction of hairiness, improvement of smoothness). The endoglucanases and endoglucanase fusion proteins of the present invention can be used in biofinishing in acid and in neutral conditions.

The endoglucanases and endoglucanase fusion proteins of the present invention are useful in detergent compositions to improve fabric care properties by antipilling, antigraying, color clarification and softening, and to improve textile cleaning effect, for instance soil removal.

Stone washing has three steps: desizing, abrasion and after-treatment. The first step, desizing process is normally the first wet treatment of jeans and means the removal of starch or other sizing agents applied usually to the warp yarns to prevent damage during the weaving process. Alpha-amylases are used to remove starch-based size for improved and uniform wet processing. After desizing the jeans are normally rinsed with water or continued directly with the abrasion step.

The second step, abrasion, can be performed with enzymes or pumice stones or both. In all cases mechanical action is needed to remove the dye, and the treatment is usually carried out in washing machines, like drum washers. The term "abraded" means herein the appearance of denim fabric when it has been treated by cellulase enzymes or stones, or both. As a result of uneven dye removal there are contrasts between dyed areas and areas from which dye has been removed. Synonymous expressions are "stone washed look" or "worn look". In enzymatic stone washing, or biostoning, abrasion with pumice stones is completely or partially eliminated and cellulase is added to facilitate the abrasion of Indigo dye from the fiber surface. The cellulase treatment may be done using neutral or acid cellulases or both.

Abrasion is generally followed by the third step, after-treatment that includes washing and rinsing steps during which detergents, optical brighteners or softeners may be used. After the enzymatic treatment the reaction must be stopped in order to prevent damage of the treated materials, for example by temperature and/or pH inactivation, the latter comprising a thorough rinsing and/or detergent wash-off. This ensures that the mechanical strength of the fiber is not further compromised by the continued presence of the enzyme.

By "denim" is meant, in connection of this invention, denim fabric, usually denim garments, particularly jeans. Advantageously the denim is Indigo dyed denim. Denim can also be treated with Indigo, with derivatives of Indigo or denim dyed with Indigo together with some other dye, for example Indigodyed denim with sulphur bottom.

Treatment with a cellulase(s) can completely replace treatment with pumice stones (for example, 1 kg commercial enzyme vs. 100 kg stones). However, cellulase treatment can be combined with pumice stone treatment when it is desired to produce a heavily abraded finish. A peach skin effect in which a fine protruding hair-like covering is created is also achieved by a wash combining a neutral cellulase with pumice stones. The cellulases of this invention are especially useful to provide abraded look and to minimize backstaining in biostoning.

Biostoning is typically performed at about pH 3.0-8.0, and preferably at pH 4.0-6.0. The temperature of the reaction can range from about 30°C to 70°C and is preferably between 50-60°C. The liquor ratio (the ratio of the volume of liquid per weight of fabric) may range from about 3:1 to 20:1, preferably 5:1 to 10:1. The treatment time can range between 15 min-90 min and preferably 30 min-60 min. It should be emphasized that the enzyme dosage depends greatly on the type of the fabrics, machinery, process conditions (pH, temperature, liquor ratio, treatment time, denim load, process scale) and type of enzyme preparation and like. If desired, pumice stones can be used in combination with the endoglucanases or endoglucanase fusion proteins. The enzyme dosage required will then be significantly lower. A person skilled in art is capable in defining suitable dosages and conditions.

The textile material that is treated with the enzyme preparations of the invention may be manufactured of natural cellulose containing fibers or manmade cellulose containing fibers or mixtures thereof. Examples of natural cellulosics are cotton, linen, hemp, jute and ramie. Examples of manmade cellulosics are viscose, cellulose acetate, cellulose triacetate, rayon, cupro and lyocell. The above-mentioned cellulosics can also be employed as blends of synthetic fibers such as polyester, polyamide or acrylic fibers. The textile material may be yarn or knitted or woven or formed by any other means.

The endoglucanases and endoglucanase fusion proteins of the present invention, besides being especially useful for the treatment of fabric, are useful in general in any area requiring cellulase activity.

In the pulp and paper industry, cellulases can be used, for example, in deinking or modifying fibre of different recycled papers and paperboards having neutral or alkaline pH, in improving the fiber quality, or increasing the drainage in paper manufacture. Other examples include the removal of printing paste thickener and excess dye after textile printing, and as a treatment for animal feed. For example, if the intended application is improvement of the strength of the mechanical pulp, then the enzyme preparations of the invention may provide one or more of these proteins so as to enhance or facilitate the ability of cellulose fibers to bind together. In a similar manner, in the application of pulp refining, the endoglucanases and endoglucanase fusion protein preparations of the invention may provide one or more of these proteins at a level that enhance or facilitate such swelling.

The endoglucanases and endoglucanase fusion proteins of the present invention provide unexpected advantages when used in textile industry and especially in biofinishing, such as depilling, and in biostoning. The endoglucanases and endoglucanase fusion proteins of the present invention are considerably more efficient than the cellulases of prior art. In biofinishing at least four-fold lower dosages could be used. In other words, higher performance is achieved by using the endoglucanases and endoglucanase fusion proteins of the present invention. In depilling the endoglucanases and endoglucanase fusion proteins of the present invention were more efficient and produced a stable smooth surface.

The invention is described in more detail in the following examples, which are not be interpreted to narrow the scope of the invention but only to clarify the use of the invention.

### Example 1. Cultivation of the Acremonium thermophilum ALKO4245

The *Acremonium thermophilum* strain ALKO4245 was grown in a 2 litre bioreactor (Braun Biostat® B, Braun, Melsungen, Germany) in the following medium, g/l: Solka Floc cellulose 40, corn steep powder 15, distiller's spent grain 5, oats spelt xylan 3, locust bean gum 3, (NH₄)₂SO₄ 5 and KH₂PO₄ 5. The pH range was 5.2±0.2 (NH₃/H₂SO₄), aeration 1 vvm, stirring 300-600 rpm, antifoam control with Struktol® and the temperature 42°C. The cultivation time was 4 days. After cultivation the cells and other solids were collected by centrifugation and the supernatant was recovered.

### Example 2. Purification of an endoglucanase from Acremonium thermophilum ALKO4245

The culture supernatant *of Acremonium thermophilum* ALKO4245, grown as described in Example 1, was incubated at 70°C for 24 hours after which it was concentrated by ultrafiltration. The pure endoglucanase was obtained by sequential purification with hydrophobic interaction and cation exchange chromatography followed by gel filtration. The endoglucanase activity of the fractions collected during purification was determined using carboxymethyl cellulose (CMC) as a substrate (according to the procedure of IUPAC, 1987).

The concentrated culture supernatant was applied to the HiPrep 16/10 Butyl FF hydrophobic interaction column (GE Healthcare) equilibrated with 20 mM potassium phosphate buffer, pH 6.0, containing 1 M (NH₄)₂SO₄. Bound proteins were eluted with a linear gradient from the above buffer to 5 mM potassium phosphate, pH 6.0. Fractions were collected and the endoglucanase activity was determined as described above. The endoglucanase activity eluted in a broad conductivity area of 120 to 15 mS/cm.

Combined fractions were applied to the HiTrap SP XL cation exchange column (GE Healthcare) equilibrated with 8 mM sodium acetate, pH 4.5. Bound proteins were eluted with a linear gradient from 0 to 0.25 M NaCl in the equilibration buffer. The protein containing endoglucanase activity eluted at the conductivity area of 3-7 mS/cm. Cation exchange chromatography was repeated and the protein eluate was concentrated by freeze drying.

The dissolved sample was loaded onto the Superdex 75 HR10/30 gel filtration column (Pharmacia) equilibrated with 20 mM sodium phosphate buffer, pH 7.0, containing 0.15 M NaCl. The main protein fraction eluted from the column with the retention volume of 13.3 ml. The protein eluate was pure as judged by SDS-polyacrylamide gel electrophoresis and the molecular weight was evaluated to be 40kDa. The specific activity of the purified protein, designated as *Acremonium thermophilum* EG_40 or At EG_40 (SEQ ID NO: 2), at 50°C was determined to be 450 nkat/mg (according to the procedure of IUPAC, 1987, *supra,* using CMC as a substrate).

The thermal stability of the purified endoglucanase was determined at different temperatures. The reaction was performed in the presence of 0.1 mg/ml BSA at pH 5.0 for 60 min using CMC as a substrate. At EG_40 was stable up to 80°C. The *T. reesei* reference enzymes EGI (Cel7B) and EGII (Cel5A) retained 100% of their activity up to 60°C and 65°C, respectively.

For internal amino acid sequencing, the purified *Acremonium thermophilum* ALKO4245 EG_40 protein (SEQ ID NO: 2) was first alkylated and digested into tryptic peptides. Generated peptides were desalted and partially separated by nano liquid chromatography (reverse-phase). The internal peptides were sequenced by electrospray ionization combined to tandem mass spectrometry (ESI-MS/MS) using the Q-TOF1 (Waters Micromass®) instrument. The internal peptide sequences so obtained are listed in Table 1.

**Table 1. Internal peptide sequences determined from the Acremonium thermophilum EG_40 cellulase**

| **Peptide** | **Sequence** | **SEQ ID NO:** |
|---|---|---|
| Peptide 1 | **QSCSSFPAPLKPGCQWR** | **5** |
| Peptide 2 | **YALTFNSGPVAGK** | **6** |
| Peptide 3 | **VQCPSELTSR** | **7** |
| Peptide 4 | **NQPVFSCSADWQR** | **8** |
| Peptide 5 | **YWDCCKPSCGWPGK** | **9** |
| Peptide 6 | **PTFT** | **10** |

### Example 3. Cloning of Acremonium thermophilum (ALKO4245) cel45A and cel45B genes

Standard molecular biology methods were used in the isolation and enzyme treatments of DNA (plasmids, DNA fragments), in *E. coli* transformations, etc. The basic methods used are described in the standard molecular biology handbooks, e.g. Sambrook, J., *et al.,* 1989 and Sambrook J. and Russell, D.W., 2001.

The genomic library of *Acremonium thermophilum* ALKO4245 was constructed in Lambda DASH^{®}II vector (Stratagene, USA) according to the instructions from the manufacturer. The chromosomal DNA, isolated by the method of Raeder and Broda, 1985, was partially digested with *Sau3A.* The digested DNA was size-fractionated in an agarose gel and the fragments of chosen size (about 5-23 kb) were isolated, dephosphorylated and ligated to the *Bam*HI digested lambda vector arms. The ligation mixture was packaged using the Gigapack III Gold packaging extracts according to the manufacturer's instructions (Stratagene, USA). The titer of the genomic library was 3.7 × 10⁵ pfu/ml and that of the amplified library was 4.2 × 10⁸ pfu/ml.

The internal peptide sequences from the purified *Acremonium thermophilum* EG_40 cellulase obtained as described in Example 2 shared homology with cellulases of the glycosyl hydrolase family 45, such as *Thielavia terrestris* endoglucanase (GenBank Accession No. CQ827970) and *Melanocarpus albomyces* Cel45A cellulase (GenBank Accession No. AJ515703). In order to amplify a probe for screening of the *A. thermophilum* EG_40 encoding gene (ce/45A; SEQ ID NO: 1) from the genomic library, degenerate primers were designed on the basis of the peptide sequences listed in Table 1 (Example 2). The order of the peptides in the protein sequence and the corresponding sense or anti-sense nature of the primers was deduced from the comparison with the homologous *M. allbomyces* Cel45A sequence. The sense primer (TAYTGGGAYTGYTGYAARCC, SEQ ID NO: 11) is based on amino acids 1 to 6 of peptide 5 (SEQ ID NO: 9) and the anti-sense primer (RTTRTCNGCRTTYTGRAACCA, SEQ ID NO: 12) is based on a peptide sequence (WFQNADN; SEQ ID NO: 13) of the homologous *M. albomyces* Cel45A protein. The PCR reaction mixtures contained 50 mM Tris-HCl, pH 9.0, 15 mM (NH₄)₂SO₄, 0.1% Triton X-100, 1.5 mM MgCl₂, 0.1 mM dNTPs, 0.5 µg of each primer, 1 unit of Dynazyme EXT DNA polymerase (Finnzymes, Finland), and approximately 0.5 µg of *Acremonium* genomic DNA. The conditions for PCR reactions were as follows: 5 min initial denaturation at 95°C, followed by 30 cycles of 1 min at 95°C, 1 min annealing at 50-60°C, 2 min extension at 72°C and a final extension at 72°C for 10 min. The extension products were examined in an agarose gel.

Two PCR products were obtained from the *Acremonium* PCR reaction. DNA fragments of about 0.6 kb (SEQ ID NO: 14) and 0.8 kb (SEQ ID NO: 15) were isolated from the agarose gel and cloned into the pCR4-TOPO^{®} TA vector (Invitrogen, USA) resulting in plasmids pALK1710 and pALK1711, respectively. The cloned PCR products were characterized by sequencing and by performing Southern blot hybridizations (as described below) to the genomic *Acremonium* DNA digested with several restriction enzymes. The hybridization patterns obtained with the two fragments in stringent washing conditions suggest that two putative endoglucanase genes could be screened from the *Acremonium* genomic library. The deduced amino acid sequences of both PCR products have homology to several published endoglucanase sequences of glycosyl hydrolase family 45 (BLAST program, National Center for Biotechnology Information; Altschul *et al.,* 1990).

The insert from plasmid pALK1710 and pALK1711 was isolated by restriction enzyme digestion and labeled with digoxigenin according to the manufacturer's instructions (Roche, Germany). About 1-2×10⁵ plaques from the amplified *Acremonium* genomic library were transferred on nitrocellulose filters and screened by hydridization using digoxigenin-labeled inserts. The temperature for hybridisation was 68°C and the filters were washed 2 x 5 min at RT using 2 x SSC-0.1% SDS followed by 2 x 15 min at 68°C using 0.1 x SSC-0.1% SDS. Several positive plaques were obtained, of which five strongly hybridizing plaques were purified from both screenings. Phage DNAs were isolated and analyzed by Southern blot hybridization. Restriction fragments of phage DNAs hybridizing to the probes were subcloned into the pBluescript II KS+ vector (Stratagene, USA) and the relevant parts were sequenced. In both cases the subcloned phage fragment contains the full-length gene of interest.

Table 2 summarizes the information of the probes used for screening of the endoglucanase genes, phage clones from which the genes were isolated, chosen restriction fragments containing the full-length genes with their promoter and terminator regions, names of plasmids containing the subcloned phage fragment, and the deposit numbers in the Deutsche Sammlung von Mikroorganismen und Zellkulturen GmbH culture collection (DSM) for *E. coli* strains carrying these plasmids. The depositions were made under the Budapest Treaty.

**Table 2. Probes used for cloning of the endoglucanase genes, phage clones and the subclones chosen, plasmid names and the corresponding deposit number of the E. coli strains**

| **Gene** | **Genomic library** | **Probe used in screening** | **Phage clone** | **Subcloned fragment** | **Plasmid** | ***E. coli* deposit no.** |
|---|---|---|---|---|---|---|
| At ce*l*45A | *A*. *thermophilum* ALKO4245 | pALK1710 | P24 | 5.5 kb Smal | pALK1908 | DSM 17324 |
| At *cel*45B | *A*. *thermophilum* ALKO4245 | pALK1711 | P41 | 6.0 kb *Xho*I | pALK1904 | DSM 17323 |

Relevant information of the two genes, designated as At *cel*45A (SEQ ID NO: 1) and At *cel*45B (SEQ ID NO: 3), is summarized in Table 3 and of the respective deduced protein sequences, At EG_40 (SEQ ID NO: 2) and At EG_40_like (SEQ ID NO: 4), in Table 4. The peptide sequences of the purified *Acremonium* EG_40 endoglucanase were found in the corresponding deduced amino acid sequence of the cloned gene confirming that an appropriate gene was cloned.

The full-length At *cel*45A gene (SEQ ID NO: 1) is 1076 bp in length, interrupted by two introns of 59 bp and 123 bp, and codes for a 297 amino acid polypeptide At EG_40 (SEQ ID NO: 2). The putative signal peptide cleavage site is after Ala21, and the N-terminus of the mature protein begins with Leu22, the mature protein (including CBD) comprising amino acids 22 to 297 of SEQ ID NO: 2). The EG_40 cellulase has a C-terminal consensus carbohydrate binding domain harboring amino acids Lys265 to Leu297 of the full-length polypeptide. The predicted mature protein after signal peptide cleavage has a molecular weight and pi of 28625 Da and 4.79, respectively (prediction made using the Compute pl/MW tool at ExPASy server, Gasteiger *et al.,* 2003). The protein has two putative N-glycosylation sites N-X-S/T (predicted using the program NetNGlyc 1.0, Gupta *et al.,* 2004).

Correspondingly, The full-length At *cel*45B gene (SEQ ID NO:3) is 1013 bp in length, interrupted by two introns of 155 bp and 102 bp, and codes for a 251 amino acid polypeptide At EG_40_like (SEQ ID NO: 4). The putative signal peptide cleavage site is after Ala20, and the N-terminus of the mature protein begins with Gln21, the mature protein comprising amino acids 21 to 251 of SEQ ID NO: 4) The EG_40_like cellulase has no C-terminal consensus carbohydrate binding domain. The predicted mature protein after signal peptide cleavage has a molecular weight and pi of 23972 Da and 6.11, respectively (prediction made using the Compute pl/MW tool at ExPASy server, Gasteiger *et al.,* 2003). The protein has two putative N-glycosylation sites N-X-S/T (predicted using the program NetNGlyc 1.0, Gupta *et al.,* 2004).

**Table 3. Summary of the endoglucanase genes isolated from Acremonium thermophilum ALKO4245**

| **Endoglucanase gene** | **Length with introns (bp) ^{(a}** | **Coding region (bp) ^{(b}** | **No of introns** | **Lengths of introns (bp)** | **SEQ ID NO:** |
|---|---|---|---|---|---|
| At *cel*45A | 1076 | 891 | 2 | 59, 123 | 1 |
| At *cel*45B | 1013 | 753 | 2 | 155, 102 | 3 |

| | | | | | |
|---|---|---|---|---|---|
| ^{(a} The STOP codon is included. ^{(b} The STOP codon is not included. | | | | | |

**Table 4. Summary of the deduced endoglucanase sequences of Acremonium thermophilum ALK04245. ss, signal sequence**

| **Endoglucanase protein** | **No of aas** | **Length of ss NN/HMM^{(a}** | **CBD^{(b}** | **Predicted MW (Da, ss not incl)^{(c}** | **Predicted pl (ss not incl)** | **Putative N-glycosylation sites^{(d}** | **SEQ ID NO:** |
|---|---|---|---|---|---|---|---|
| At EG_40 | 297 | 21/21 | Yes; K265 to L297 | 28625 | 4.79 | 2 | 2 |
| At EG_40_like | 251 | 20/20 | No | 23972 | 6.11 | 2 | 4 |

| | | | | | | | |
|---|---|---|---|---|---|---|---|
| ^{(a} The prediction on the signal sequence was made using the program SignalP V3.0 (Nielsen *et al.,* 1997; Bendtsen *et al.,* 2004); the NN value was obtained using neural networks and HMM value using hidden Markov models. ^{(b} Presence of a carbohydrate binding domain in the protein, the amino acids of the C-terminal CBD are indicated (numbering according to the full length polypeptide) ^{(c} The predicted signal sequence is not included. Prediction was made using the Compute pl/MW tool at ExPASy server (Gasteiger *et al.,* 2003). ^{(d} The putative N-glycosylation sites N-X-S/T were predicted using the program NetNGlyc 1.0 (Gupta *et al.,* 2004, In preparation; www.cbs.dtu.dk/services/NetNGlyc/). | | | | | | | |

The deduced protein sequences of the *A. thermophilum* EG_40 and EG_40_like cellulases are similar to cellulases of glycosyl hydrolase family 45 (Table 5). The closest sequence homologies found for EG_40/Cel45A and EG_40_like/Cel45B were endoglucanase sequences of *Thielavia terrestris* (GenBank Accession No. CQ827970) and *Myceliophthora thermophila* (GenBank Accession No. AR094305), respectively. The alignments were performed using the Needle programme of the EMBOSS programme package.

**Table 5. Comparison of the deduced protein sequences of the Acremonium thermophilum EG_40 and EG_40_like cellulases with their homologous counterparts**

| **Organism, enzyme, and accession number** | **Identity (%)** |
|---|---|
| ***Acremonium thermophilum* EG_40** | |
| *Thielavia terrestris* EG45, CQ827970 | 77.3 |
| *Melanocarpus albomyces* Cel45, AJ515703 | 75.3 |
| *Neurospora crassa,* hypothetical XM_324477 | 68.9 |
| *Humicola risea* var *thermoidea,* EGL3, AB003107 | 67.5 |
| *Humicola insolens* EG5, A23635 | 67.3 |
| *Myceliophtora thermophila* fam 45, AR094305 | 57.9 |
| ***Acremonium thermophilum* EG_40_like** | 53.7 |
| ***Acremonium thermophilum* EG_40_like** | |
| *Myceliophtora thermophila* fam 45, AR094305 | 66.9 |
| *Magnaporthe grisea* 70-15 hypothetical, XM_363402 | 61.9 |
| *Thielavia terrestris* EG45, CQ827970 | 56.8 |
| ***Acremonium thermophilum* EG_40** | 53.7 |
| *Melanocarpus albomyces* Cel45, AJ515703 | 52.8 |

### Example 4. Production of Acremonium thermophilum EG_40 and EG_40_like cellulases in Trichoderma reesei

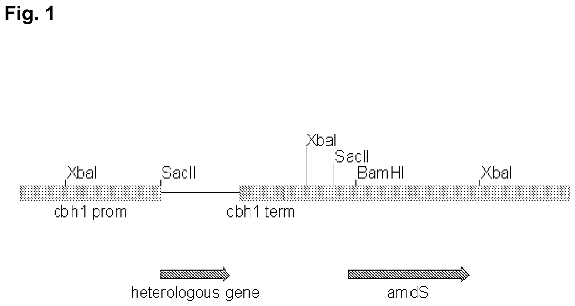

Expression plasmids were constructed for production of the recombinant *A. thermophilum* EG_40/Cel45A and EG_40_like/Cel45B cellulases. Both genes (*cel*45A or *cel*45B), including their own signal sequence, were exactly fused to the *T.reesei cbh1 (cel7A)* promoter by PCR (Table 6). The *cbh1* promoter, *cbh1* terminator and *amdS* marker gene were included as described in Paloheimo *et al.* 2003, *supra.* The linear expression cassette (Figure 1) was isolated from the vector backbone by restriction enzyme digestion, transformed into *T. reesei* A96, and transformants selected with acetamide as sole nitrogen source. The host strain lacks four major endogenous cellulases: CBHI/Cel7A, CBHII/Cel6A, EGI/Cel7B and EGII/Cel5A. The transformations were performed according to Penttilä *et al,* 1987, with the modifications described in Karhunen *et al.,* 1993. The transformants were purified on selection plates through single conidia prior to sporulating them on potato extract agar.

**Table 6. The expression cassettes constructed for production of Acremonium thermophilum EG_40 and EG_40_like cellulases in Trichoderma reesei. The schematic structure of the expression cassettes is described in Figure 1**

| **Endoglucanase** | **Expression plasmid** | **Size of the expression cassette^{(a}** | **Heterologous terminator^{(b}** |
|---|---|---|---|
| At EG_40 | pALK1920 | 10.9 kb *Not*I | 156 bp (*Hin*dIII) |
| At EG_40_like | pALK1921 | 8.6 kb *Eco*RI | 282 bp (*Ssp*I) |

| | | | |
|---|---|---|---|
| ^{(a} The expression cassette for *T. reesei* transformation was isolated from the vector backbone by *Eco*RI or *Not*I digestion. ^{(b} The number of nucleotides after the STOP codon of the cloned gene that are included in the expression cassette are indicated. The restriction site at the 3'-region of the gene that was used in construction of the expression cassette is indicated in parenthesis. | | | |

The endoglucanase production of the transformants was analyzed from the culture supernatants of shake flask cultivations (50 ml). The transformants were grown for 7 days in a complex cellulose-inducing medium (Joutsjoki *et al.,* 1993) buffered with 5% KH₂PO₄ at pH 5.5. The enzyme activity of the recombinant protein was measured from the culture supernatant as the release of reducing sugars from carboxymethylcellulose (2% CMC) at 50°C in 50 mM Sitrate buffer pH 4.8 essentially as described by Bailey, M. J. and Nevalainen, K.M.H., 1981; Haakana, H., *et al,* 2004. Production of the recombinant protein was also detected from the culture supernatant by SDS-polyacrylamide gel electrophoresis. EG_40-specific polyclonal antibodies were produced in rabbits (University of Helsinki, Finland). The expression of EG_40 cellulase was verified by Western blot analysis with anti-EG_40 antibodies using the ProtoBlot Western blot AP system (Promega). The genotypes of the chosen transformants were analysed by Southern blotting using the expression cassette as a probe.

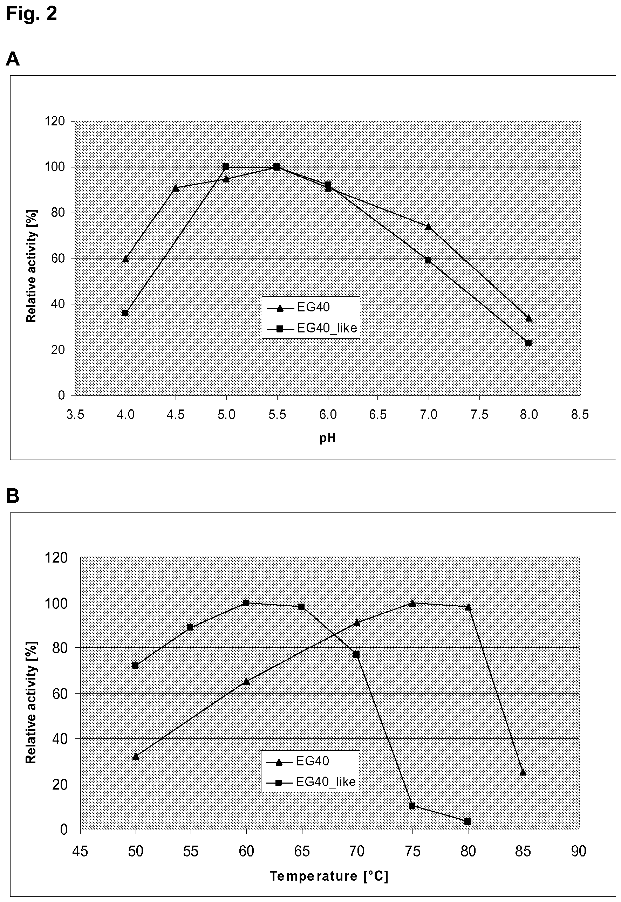

The pH optimum of the heterologously produced EG_40/Cel45A and EG_40_like/Cel45B cellulases was determined in the universal Mcllvaine's buffer within a pH range of 4.0-8.0 using CMC as a substrate. As shown in Figure 2A, the pH range of EG_40/Cel45A cellulase is relatively broad (4.5-6.0), the optimum being at pH 5.5. The pH optimum for EG_40_like/Cel45B was determined to be pH 5.0-5.5. The optimal temperature for enzymatic activity of EG_40/Cel45A and EG_40_like/Cel45B cellulases was determined to be 75-80°C and 60°C, respectively (Figure 2B). The thermal stability of the heterologously produced EG_40/Cel45A cellulase is comparable to that of the purified protein.

The chosen transformants RF6118 (At EG_40) and RF6071 (At EG_40_like) were cultivated in a 2 litre bioreactor for four days (28°C, pH 4.2) to obtain material for the application tests (see Examples 8 to 13).

### Example 5. Construction and production of modified Acremonium thermophilum EG_40 cellulases

Standard molecular biology methods were used as described in Example 3. The signal sequence of EG_40 cellulase was replaced with that of *Trichoderma reesei* CBHI. The use of the host's endogeneous signal sequence was expected to improve the heterologous protein production. In order to amplify the 5' fragment of the At *cel*45A gene two oligonucleotides were designed. The sense primer (ATTAACCGCGGACTGCGCATCATGTATCGGAAGTTGGCCGTCATCTCGG CCTTCTTGGCCACAGCTCGTGCCCTCGACGGAAAGTCGAC, SEQ ID NO: 22) contains the signal sequence of *T. reesei cel*7A and the anti-sense primer (TCGACTGCACCACCATGGTC, SEQ ID NO: 23) is At ce*l*45A-specific. The full-length gene was reconstituted by ligation of the amplified PCR product as a *Sac*II-*Nco*I fragment with the 3'-end of the At *cel*45A gene.

Subsequently, the carbohydrate binding domain of EG_40/Cel45A was modified: Three constructs were prepared containing the catalytic domain of EG_40/Cel45A (amino acids 22-234 of SEQ ID NO: 2) attached to the linker region and CBD of either *Trichoderma reesei* CBHI/Cel7A (SEQ ID NO: 24), *Acremonium thermophilum* ALKO4245 XYN60/Xyn10A (SEQ ID NO: 25), or *Chaetomium thermophilum* ALKO4265 CBHI/Cel7A (SEQ ID NO: 26). The linker and CBD region of the *T. reesei* CBHI/Cel7A encoding gene (*cel*7A, SEQ ID NO: 27), *A. thermophilum* ALKO4245 XYN60/Xyn10A encoding gene *(xyn10A,* SEQ ID NO: 28), or C. *thermophilum* ALKO4265 CBHI/Cel7A encoding gene (*cel*7A, SEQ ID NO: 29) was amplified by PCR using oligonucleotide combinations of TTGGATCCGAGTCGCAGCGGC-AACCCTAG-CGGCGGCAAC (SEQ ID NO: 30) + TAATTCTGCA-GTTACAGGCACTGAGAGTAG (SEQ ID NO: 31), or TTGGATCCGA-GTCGCAGCGGCGGGAACCCACCCCCCGTCAC (SEQ ID NO: 32) + TAATTCTGCAGTCACAGGCACTGAGAGTACCAGT (SEQ ID NO: 33), or TAATTTACGTACCTGGCCTTGACGGCAG (SEQ ID NO: 34) + ATTAACTGCAGTTACAGGCACTGTTGAGCA (SEQ ID NO: 35), respectively. The amplified PCR products were ligated to the *cel*45A gene to create At *cel*45A_Tr *cel*7AlinkerCBD (SEQ ID NO: 36), At *cel*45A_At *xyn*10AlinkerCBD (SEQ ID NO: 37), or At *cel*45A_Ct *cel*7AlinkerCBD (SEQ ID NO: 38) genes. The resulting plasmids were designated as pALK2022, pALK2024, and pALK2026. The deposit numbers of the corresponding *E. coli* strains in the Deutsche Sammlung von Mikroorganismen und Zellkulturen GmbH culture collection are DSM 18815, DSM 18816, and DSM 18817, respectively. The depositions were made under the Budapest Treaty.

Expression plasmids for production of modified EG_40/Cel45A proteins were constructed and the recombinant proteins EG_40_TrCBD (SEQ ID NO: 39), EG_40_AtCBD (SEQ ID NO: 40), and EG_40_CtCBD (SEQ ID NO: 41) were produced in *Trichoderma* as described in Example 4. The temperature and pH optimum of the modified EG_40/Cel45A cellulases was similar with that of the wild-type protein. The chosen transformants RF6828 (EG_40_TrCBD), RF6835 (EG_40_AtCBD), and RF6821 (EG_40_CtCBD) were cultivated in a 2 litre bioreactor for four days (28°C, pH 4.2) to obtain material for the application tests (Examples 9 and 13).

### Example 6. Construction and production of Acremonium thermophilum ALKO4245 EG_40 cellulases lacking carbohydrate binding domain or carbohydrate binding domain plus the linker region

To produce *Acremonium thermophilum* ALKO4245 EG_40 cellulases lacking carbohydrate binding domain (CBD), At cel45A_CBDless, or the CBD plus the linker region, At cel45A_linkerCBDless, two *cel*45A deletion constructs were made; the first lacking the region coding for the CBD and the second additionally lacking the linker region between the catalytic core and the carbohydrate binding domain.

Standard molecular biology methods were used as described in Example 3. Two different length 3'-fragments of the ce/45A gene were amplified by PCR. The antisense primers were designed to exclude the CBD or the linker+CBD region from the product. A PCR product amplified with primers GCAGCAACCAGTTCGACCTC (SEQ ID NO: 42) and TTAACTGCAGTCACTGGGCAGTGCAGCCACCGCCTC (SEQ ID NO: 43) was ligated as a *Sac*I*-Pst*I fragment and another PCR fragment amplified with primers ACTGCTGCAAGCCGTCCTGC (SEQ ID NO: 44) and TTAACTGCAGTCAACCGCTAGGCGGGTTGAAGACGGGATAG (SEQ ID NO: 45) as an *Nco*I*-Pst*I fragment to the 5'fragment of the *cel*45A gene to reconstitute full-length genes At *cel*45A_CBDless (SEQ ID NO: 16) and At *cel*45A_linkerCBDless (SEQ ID NO: 18), respectively. The resulting plasmids were designated as pALK2009 and pALK2014. The deposit numbers of the corresponding *E. coli* strains in the Deutsche Sammlung von Mikroorganismen und Zellkulturen BmbH culture collection are DSM 18813, and DSM 18814, respectively. The depositions were made under the Budapest Treaty.

Expression plasmids for the production of the CBDless and link-erCBDless versions of *A. thermophilum* EG_40/Cel45A cellulase were constructed and the recombinant proteins (SEQ ID NO: 17 and 19, respectively) were produced in *Trichoderma* as described in Example 4.

### Example 7. Construction and production of the recombinant Acremonium thermophilum ALKO4245 EG_40_like+CBD fusion protein

To production of a recombinant *Acremonium thermophilum* ALKO4245 EG_40_like+CBD fusion protein (SEQ ID NO: 21), the carbohydrate binding domain (CBD) of the EG_40/Cel45A cellulase is linked to the EG_40_like cellulase. The construct contains the catalytic domain of EG_40_like (amino acids 1-242 of the full-length polypeptide) attached to the linker region and CBD of EG_40 cellulase (amino acids 235-297 of the full-length polypeptide).

Standard molecular biology methods are used as described in Example 3. First, a unique *Nru*I restriction site near the C-terminal end of the EG_40_like sequence is introduced by PCR. This enables direct fusion of any blunt-ended DNA after amino acid S242 of the EG_40_like polypeptide. The linker+CBD region of the EG_40 encoding gene (*cel*45A) is amplified by PCR and a restriction fragment thereof ligated to the *cel*45B gene (after S242) to create At *cel*45B_*cel*45AlinkerCBD (SEQ ID NO: 20). Expression plasmid for production of the EG_40_likeCBD cellulase is constructed and the recombinant protein (SEQ ID NO: 21) produced in *Trichoderma* as described in Example 4.

### Example 8. Performance of EG_40 cellulase preparation in denim finishing at different temperatures

*Acremonium thermophilum* EG_40 cellulase from strain RF6118 produced using *Trichoderma reesei* as host as described in Example 4 was tested for its ability to create abraded look similar to that provided by pumice stones in biostoning of denim at different temperatures. A commercial EGII enriched preparation produced using *Trichoderma* as host (US 5,874,293) efficient in denim finishing was used for comparison at 50°C.

Jeans made of Indigo dyed denim twill were used as test material after desizing with ECOSTONE^{®} A200 alpha-amylase. The cellulase treatments were performed with Electrolux's Wascator FOM 71 CLS washer extractor under conditions described in Table 7.

The EGII enriched stabilized enzyme concentrate was dosed at 0.23% on the weight of the fabric, which is a typical dosage for the preparation in industrial applications. A concentrated and stabilized EG_40 preparation obtained from pilot fermentation was dosed at 0.18%. When calculated [as protein] in terms of the protein content, the dosages were ca. 0.20 mg and 0.035 mg per g of fabric using the Bio-Rad Protein Assay Dye Reagent (BioRad, Hercules, CA, USA) and bovine gammaglobulin as the standard. The cellulase enzyme was inactivated after draining by raising the pH above 11 through an addition of 5 g of NaOH (10 min. 40°C) and rinsing three times. The jeans were dried in a tumbler.

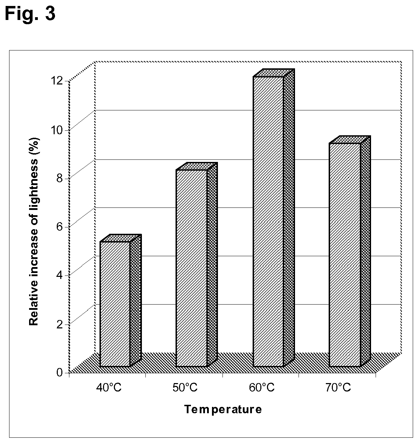

The biostoning effect/abrasion level was evaluated by measuring the color as reflectance values with Minolta CM 2500 spectrophotometer using L*a*b* color space coordinates (illuminant D65/2°). The color from the face side and the reverse side of denim was measured after desizing (i.e. before the cellulase treatment) and after the cellulase treatment. Each measurement value on the face side of denim was an average of approximate 40 measurements. Two pairs of jeans were used in each test and the final result was the average of them. The results are shown in Table 8 and Fig. 3.

**Table 7. The test conditions/process parameters used in cellulase treatments**

| **Process parameter** | |
|---|---|
| Denim load | 1.3-1.4 kg |
| Water | 19 liter |
| pH control (pH 5-5.3) | 5 ml Acetic acid (80%) |
| Time | 45 min |
| Temperature | 40, 50, 60, or 70°C |
| Cellulase dosage | 0.18% or 0.23% on the weight of the fabric |

**Table 8. Color measurements of the face side of denim treated with EG_40 preparation at different temperatures**

| **Enzyme preparation** | **Dosage, % owf^{a)}** | **Protein, mg/ g fabric** | **Temp., °C** | **Before cellulase treatment** | | **After cellulase treatment** | | **Increase** |
|---|---|---|---|---|---|---|---|---|
| | | | | **L*** | **b*** | **L*** | **b*** | **of L*** |
| EGII enriched conc. | 0.23 | 0.20 | 50 | 22.24 | -15.57 | **30.08** | -17.64 | **7.84** |
| EG_40 conc. | 0.18 | 0.035 | 70 | 21.88 | -15.54 | 31.07 | -16.76 | 9.20 |
| EG_40 conc. | 0.18 | 0.035 | 60 | 22.21 | -15.40 | 34.16 | -16.34 | 11.95 |
| EG_40 conc. | 0.18 | 0.035 | 50 | 22.00 | -15.22 | **30.10** | -17.26 | **8.10** |
| EG_40 conc. | 0.18 | 0.035 | 40 | 22.13 | -14.98 | 27.26 | -17.50 | 5.13 |

| | | | | | | | | |
|---|---|---|---|---|---|---|---|---|
| a) on the weight of the fabric Treatment with EGII enriched preparation was used for comparison at 50°C. L* indicates the lightness, -b* is the blue direction, +b* is the yellow direction. | | | | | | | | |

Results in Table 8 and Fig. 3 show that the biostoning effect of EG_40 was very good at a low dosing range. With the EG_40 preparation similar abrasion level (lightness L*) compared to EGII enriched preparation was obtained at 50 °C with a 6 times lower amount of protein.

### Example 9. Performance of modified EG_40 cellulases in denim finishing

Modified *Acremonium thermophilum* EG_40 cellulases produced in *Trichoderma reesei* as described in Example 5 were tested for their ability to create abraded look similar to that provided by pumice stones in biostoning of denim. Recombinant proteins EG_40_TrCBD, EG_40_AtCBD, and EG_40_CtCBD were compared to wild-type EG_40 cellulase (Example 4).

Denim legs made of Indigo dyed denims of different type were used as test material after desizing with ECOSTONE^{®} A200 alpha-amylase. The cellulase treatments were performed with Electrolux's Wascator FOM 71 CLS washer extractor under conditions described in Table 9.

Cellulase preparations were dosed at 200 nkat/g on the weight of the fabric. The endoglucanase activity was measured as in Example 4, except that 3% CMC and 60°C was used. The cellulase enzyme was inactivated after draining by raising the pH above 11 through an addition of 4.2 g of NaOH (10 min. 40°C) and rinsing three times. The denim legs were dried in a tumbler.

The biostoning effect/abrasion level was evaluated by measuring the color on the face side of denim as in Example 8. Each measurement value on the face side of denim leg was an average of approximate 20 measurements. The final result was the average of three different denims from Ukos Sport, Belgium (Intrique, Atlanta, Nostalgy). The results are shown in Table 10.

**Table 9. The test conditions/process parameters used in cellulase treatments**

| **Process parameter** | |
|---|---|
| Denim load | 1.1 kg |
| Water | 17 liter |
| pH control (pH 5) | 27 g Na₂HPO₄2H₂O + 19 g citric acid |
| Time | 55 min |
| Temperature | 60°C |
| Cellulase dosage | 200 nkat/g fabric |

**Table 10. Color measurements of the face side of denim treated with different EG_40 cellulases at 60°C and pH 5**

| **Cellulase** | **Denim** | **Dosage nkat/g ^{a)}** | **Before cellulase treatment ^{b)}** | | **After cellulase treatment ^{b)}** | | **Increase of L*** |
|---|---|---|---|---|---|---|---|
| | | | **L*** | **b** | **L*** | **b** | |
| **EG_40_TrCBD** | Intrigue | | 20.56 | -9.88 | 26.43 | -12.22 | 5.87 |
| | Atlanta | | 21.22 | -16.41 | 25.99 | -18.40 | 4.77 |
| | Nostalgy | | 19.70 | -10.26 | 24.49 | -13.11 | 4.79 |
| | **Average** | 200 | **20.49** | **-12.18** | **25.64** | **-14.58** | **5.14** |
| **EG_40_AtCBD** | Intrigue | | 20.63 | -10.01 | 27.08 | -12.61 | 6.45 |
| | Atlanta | | 21.26 | -16.6 | 27.11 | -18.41 | 5.85 |
| | Nostalgy | | 19.56 | -10.08 | 24.18 | -13.24 | 4.62 |
| | **Average** | 200 | **20.48** | **-12.23** | **26.12** | **-14.75** | **5.64** |
| **EG_40_CtCBD** | Intrigue | | 20.58 | -9.91 | 26.97 | -11.94 | 6.39 |
| | Atlanta | | 21.19 | -16.45 | 26.61 | -18.06 | 5.42 |
| | Nostalgy | | 19.70 | -10.17 | 23.71 | -12.53 | 4.01 |
| | **Average** | 200 | **20.49** | **-12.18** | **25.76** | **-14.18** | **5.27** |
| **EG_40** | Intrigue | | 20.59 | -9.76 | 26.56 | -12.24 | 5.97 |
| | Atlanta | | 21.26 | -16.59 | 26.83 | -18.52 | 5.57 |
| | Nostalgy | | 19.61 | -10.12 | 24.32 | -13.09 | 4.71 |
| | **Average** | 200 | **20.49** | **-12.16** | **25.90** | **-14.62** | **5.42** |

| | | | | | | | |
|---|---|---|---|---|---|---|---|
| ^{a)} on the weight of the fabric ^{b)} L* indicates the lightness, -b* is the blue direction, +b* is the yellow direction. | | | | | | | |

The results in Table 10 show that the biostoning effect of the modified EG_40 preparations was comparable to that obtained by using the EG_40 preparation containing wild type cellulase.

### Example 10. Boosting the washing performance of EGII enriched enzyme preparation with EG_40 cellulase in denim finishing

The effect of EG_40 cellulase with EGII enriched preparation was tested in biostoning of denim. The denim and test system for biostoning were as in Example 8, except for the temperature, which was 50°C. Also the effect of the cellulase treatment was evaluated as in Example 8. Enzyme preparations were dosed at 3-5 grams resulting in 0.22-0.38% on the weight of the fabric (Table 11).

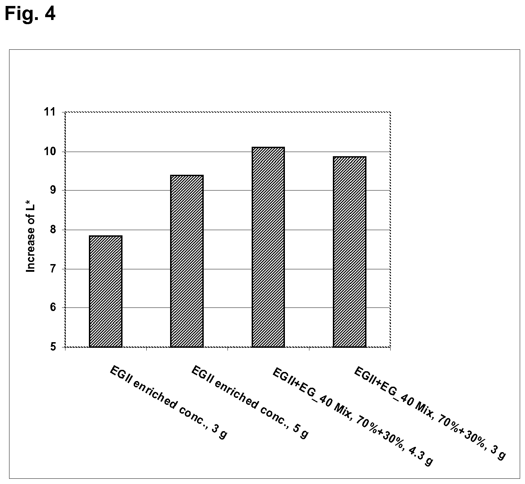

The results in Table 11 and Fig. 4 show that EG_40 can also be used to improve the abrasion effect of an EGII enriched preparation. With the EGII enriched preparation alone similar lightness levels to those obtained by the mixture containing 70% of the EGII enriched concentrate and 30% of EG_40 cellulase concentrate could not be obtained even with an increased dosage.

**Table 11. Color measurements of the face side of denim treated at 50 °C with mixture of EGII enriched and EG_40 preparations compared to EGII enriched alone**

| **Enzyme preparation** | **Dosage, g** | **Dosage, % owf^{a)}** | **Before cellulase treatment ^{b)}** | | **After cellulase treatment ^{b)}** | | **Increase of lightness** |
|---|---|---|---|---|---|---|---|
| | | | **L*** | **b*** | **L*** | **b*** | |
| EGII enriched conc. | 5 | 0.38 | 22.32 | -15.47 | 31.72 | -17.50 | 9.40 |
| EGII enriched conc. | 3 | 0.23 | 22.24 | -15.57 | 30.08 | -17.64 | 7.84 |
| EGII+EG_40 mix. 70%+30% | 4.3 | 0.33 | 22.20 | -15.53 | 32.31 | -17.46 | 10.11 |
| EGII+EG_40 mix 70%+30% | 3 | 0.22 | 22.18 | -15.74 | 32.03 | -17.55 | 9.85 |

| | | | | | | | |
|---|---|---|---|---|---|---|---|
| a) on the weight of the fabric b) L* indicates the lightness, -b* is the blue direction, +b* is the yellow direction. | | | | | | | |

### Example 11. Performance of EG_40_like cellulase preparation in denim finishing

EG_40_like fermentation liquid from strain RF6071 produced as described in Example 4 was compared to a EGII enriched concentrate in biostoning of denim. The denim and test system for biostoning were as in Example 8, except for the temperature, which was 60°C and the amount of denim, which was leveled to 1430 g with an extra piece of different denim that was not included in the measurements. Also the effect of the cellulase treatment was evaluated as in Example 8.

The results in Table 12 show that the abrasion effect of EG_40_like was obtained with less back-staining (re-deposition of Indigo-dye) on the reverse side of denim. Especially the lightness of the pockets was higher and they were less blue.

**Table 12. Color measurements of the face and reverse side of denim and pockets treated at 60°C with EG_40 _like preparation**

| **Enzyme preparation** | **Dosage** | **Prot., mg/ g fabric** | **Before cellulase treatment** | | **After cellulase treatment** | | **deltaL*** | **deltab*** |
|---|---|---|---|---|---|---|---|---|
| | | | **L*** | **b*** | **L*** | **b*** | | |
| **Face side:** | | | | | | | | |
| EG_40_like | 100 ml | 0.32 | 23.78 | -16.20 | **31.09** | -17.36 | **7.31** | -1.16 |
| EGII enriched | 1.5 g | 0.095 | 23.64 | -16.28 | **31.09** | -17.39 | **7.45** | -1.11 |

| **Reverse side:** | | | | | | | | |
|---|---|---|---|---|---|---|---|---|
| EG_40_like | 100 ml | 0.32 | 49.24 | -7.34 | 47.74 | -11.39 | **-1.50** | -4.05 |
| EGII enriched | 1.5 g | 0.095 | 49.32 | -6.97 | 47.24 | -11.78 | **-2.09** | -4.81 |

| **Pockets:** | | | | | | | | |
|---|---|---|---|---|---|---|---|---|
| EG_40_like | 100 ml | 0.32 | 75.42 | -8.78 | 66.39 | -13.20 | **-9.03** | -4.42 |
| EGII enriched | 1.5 g | 0.095 | 76.63 | -7.95 | 64.41 | -13.91 | **-12.22** | -5.96 |

| | | | | | | | | |
|---|---|---|---|---|---|---|---|---|
| Treatment with EGII enriched preparation was used for comparison. L* indicates the lightness, -b* is the blue direction, +b* is the yellow direction. | | | | | | | | |

### Example 12. Performance of EG_40 and EGII enriched preparation boosted with EG_40 in biofinishing (depilling)

The ability of the concentrated RF6118 EG_40 preparation and the ability of EGII enriched preparation boosted with EG_40 in depilling of cotton knitwear were compared to a commercial EGII enriched preparation, typically used in biofinishing formulations. The cellulase treatments were performed with Electrolux's Wascator FOM 71 CLS washer extractor under conditions described in Table 13.

Pieces of two kinds of low quality blue Polo-neck sweaters with fuzzy surface, made of 100 % cotton jersey-based fabric or rib made of 95% cotton and 5% lycra, were used as test material. Filling material was added up to 1 kg. Samples were first pre-washed for 10 min at 60°C with 1 ml/l surfactants/wetting agents (Sandoclean PCJ from Sandos and Imacol CN from Clariant) and rinsed 3 times. After this the cotton knits were treated with cellulase at 60°C for 60 minutes in the presence of the same textile auxiliaries as used in pre-wash. The enzyme was inactivated as described in Example 8, except for the temperature which was 60°C during the alkaline rinse, and the pieces of knitwear were rinsed three times and dried in the dumbler.

**Table 13. The test conditions/process parameters used in biofinishing treatments**

| **Process parameter** | |
|---|---|
| Fabric load | 1.0 kg |
| Water | 15 liter |
| Sandoclean PCJ and Imacol CN | 1 ml/l |
| Buffer/pH control (pH 5-5.3) | ca. 3 ml Acetic acid (80%) |
| Time | 60 min |
| Temperature | 60°C |
| Cellulase dosage | 0.04% to 0.63% on the weight of the fabric |

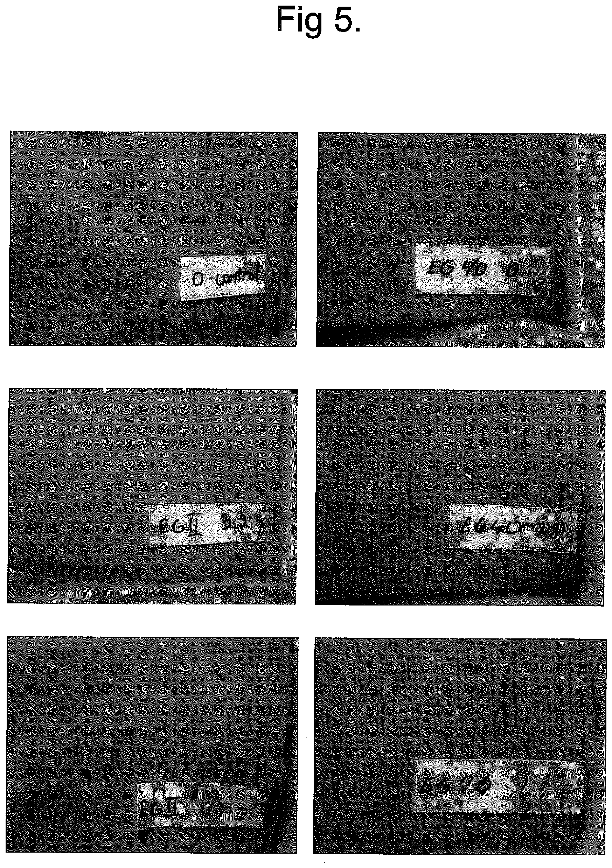

The effect of the cellulase treatment was evaluated visually with a naked eye and with a loupe. Pre-washed sample without enzyme was used as control. The results are shown in Table 14 and digital camera photos taken with a macroobjective are shown in Fig. 5.

The EG_40 preparation and the EGII enriched preparation boosted with EG_40 had excellent depilling properties compared to the commercial EGII enriched preparation that was used at dosing range typical for this enzyme concentrate in the biofinishing application. With the EG_40 preparation at least 8 times lower dosage and with the EGII enriched-EG_40 mixture at least 4 times lower dosages could be used than with EGII preparation to obtain similar effect.

The protein levels in the bioreactor culture supernatant are somewhat lower with the RF6118 strain than the *Trichoderma* producer strain of the EGII enriched preparation, when assayed with the used protein determination assay. In spite of this, the EG_40 culture medium is volumetrically at least 4-6 times more effective in biofinishing.

**Table 14. The results of biofinishing treatments with EG_40 and EGII enriched preparations boosted with EG_40 compared to EGII enriched alone**

| **Sample** | **Dosage g** | **Dosage, % owf^{a)}** | **Depilling effect^{b)}** | **Prot mg/g fabric** |
|---|---|---|---|---|
| EGII enriched conc. | 6.3 | 0.63 | + + + + + | 0.55 |
| EGII enriched conc. | 3.2 | 0.32 | + + + | 0.27 |
| EGII enriched+ EG_40 mix. 70%+13% | 3.2 | 0.32 | + + + + + | 0.21 |
| EGII enriched+ EG_40 mix. 70%+13% | 1.6 | 0.16 | + + + + + | 0.10 |
| EGII enriched+ EG_40 mix. 70%+13% | 0.8 | 0.08 | + + + | 0.052 |
| EG40 conc. | 1.6 | 0.16 | + + + + + | 0.050 |
| EG_40 conc. | 0.8 | 0.08 | + + + + + | 0.025 |
| EG_40 conc. | 0.4 | 0.04 | + + + | 0.012 |
| Prewashed only, without enzyme | - | - | - | - |

| | | | | |
|---|---|---|---|---|
| ^{a)} on the weight of the fabric ^{b)} + + + + + Excellent depilling effect, visually very clean surface + + + Good depilling effect, visually relatively clean surface - Dense surface fuzzing/and or severe pilling | | | | |

### Example 13. Performance of modified EG_40 cellulases in biofinishing (depilling)

Modified *Acremonium thermophilum* EG_40 cellulases produced in *Trichoderma reesei* as described in Example 5 were tested for their ability in depilling of cotton knitwear. Recombinant proteins EG_40_TrCBD, EG_40_AtCBD, and EG_40_CtCBD were compared to wild-type EG_40 cellulase. The cellulase treatments were performed with Electrolux's Wascator FOM 71 CLS washer extractor under conditions described in Example 12, except that the enzyme was dosed at 83 nkat/g fabric. Endoglucanase activity was measured as described in Example 9.

Pieces of three different knitwears with fuzzy surface, made of 100% cotton or 95% cotton and 5% lycra, were used as test material. Filling material was added up to 1 kg. Samples were treated as described in Example 12. The effect of the cellulase treatment was evaluated visually with a naked eye and with a loupe. Pre-washed sample without enzyme was used as control. The results are shown in Table 15.

All EG_40 preparations had excellent depilling properties leading to extensive reduction in fuzzing and prevention of the pill formation. Control samples, treated without enzyme contained dense surface fuzzing and severe pilling.

The preliminary tests performed at 50°C and 30 min using enzyme dosage of 42 nkat/g fabric showed that all of the different EG_40 cellulases had considerable depilling effect also, when lower dosages and/or short reaction time is used.

**Table 15. The results of biofinishing treatments with different EG_40 cellulases**

| **Sample** | **Dosage, nkat/g fabric** | **Depilling effect** |
|---|---|---|
| EG_40_TrCBD | 83 | + + + + + |
| EG_40_AtCBD | 83 | + + + + + |
| EG_40_CtCBD | 83 | + + + + + |
| EG_40 | 83 | + + + + + |
| Washed without enzyme | - | - |

| | | |
|---|---|---|
| + + + + + Excellent depilling effect, visually very clean surface - Dense surface fuzzing/and or severe pilling | | |

### List of deposited organisms

*Acremonium thermophilum* ALKO4245 was deposited at the Centralbureau Voor Schimmelcultures at Uppsalalaan 8, 3584 CT, Utrecht, the Netherlands (CBS) on Sep 20, 2004 under CBS 116240.
*Escherichia coli* containing plasmid pALK1908 was deposited at Deutsche Sammlung von Mikroorganismen und Zellkulturen GmbH (DSM), Mascheroder Weg 1 b, D-38124 Braunschweig, Germany on May 13, 2005 under DSM 17324.
*Escherichia coli* containing plasmid pALK1904 was deposited at Deutsche Sammlung von Mikroorganismen und Zellkulturen GmbH (DSM), Mascheroder Weg 1 b, D-38124 Braunschweig, Germany on May 13, 2005 under DSM 17323.
*Escherichia coli* containing plasmid pALK2009 was deposited at Deutsche Sammlung von Mikroorganismen und Zellkulturen GmbH (DSM), Mascheroder Weg 1 b, D-38124 Braunschweig, Germany on November 24, 2006 under DSM 18813.
*Escherichia coli* containing plasmid pALK2014 was deposited at Deutsche Sammlung von Mikroorganismen und Zellkulturen GmbH (DSM), Mascheroder Weg 1 b, D-38124 Braunschweig, Germany on November 24, 2006 under DSM 18814.
*Escherichia coli* containing plasmid pALK2022 was deposited at Deutsche Sammlung von Mikroorganismen und Zellkulturen GmbH (DSM), Mascheroder Weg 1 b, D-38124 Braunschweig, Germany on November 24, 2006 under DSM 18815.
*Escherichia coli* containing plasmid pALK2024 was deposited at Deutsche Sammlung von Mikroorganismen und Zellkulturen GmbH (DSM), Mascheroder Weg 1 b, D-38124 Braunschweig, Germany on November 24, 2006 under DSM 18816.
*Escherichia coli* containing plasmid pALK2026 was deposited at Deutsche Sammlung von Mikroorganismen und Zellkulturen GmbH (DSM), Mascheroder Weg 1 b, D-38124 Braunschweig, Germany on November 24, 2006 under DSM 18817.

### References

Altschul SF, W Gish, W Miller, EW Myers and DJ Lipman. 1990. Basic local alignment search tool. J. Mol. Biol. 215:403-410.
Bailey MJ and KMH Nevalainen 1981. Induction, isolation and testing of stable Trichoderma reesei mutants with improved production of solubilizing cellulase. Enz Microbiol Technol. 3: 153-157.
Bendtsen JD, H Nielsen, G von Heijne and S Brunak. 2004. Improved prediction of signal peptides: SignalP 3.0. J. Mol.Biol. 340:783-795.
Gasteiger, E, A Gattiker, C Hoogland, I Ivanyi, RD Appel and A Bairoch. 2003. ExPASy: the proteiomics server for in-depth protein knowledge and analysis. Nucleic Acids Res. 31:3784-3788.
Gupta, R., E. Jung and S. Brunak. 2004. Prediction of N-glycosylation sites in human proteins, In preparation. www.cbs.dtu.dk/services/NetNGlyc/
Nierstrasz V.A. and Warmoeskerken M.M.C.G. (2003) Process engineering and industrial enzyme applications. In: Textile processing with enzymes. A.Cavaco-Paulo and G.M.Gübitz (eds.) Woodhead Publishing Ltd, Cambridge. pp.120-157.
Haakana H, A Miettinen-Oinonen, V Joutsjoki, A Mäntylä, P Suominen, and J Vehmaanperä. 2004. Cloning of cellulase genes from Melanocarpus albomyces and their efficient expression in Trichoderma reesei. Enz Microbiol Technol. 34: 159-167.
Henrissat B. (1991) A classification of glycosyl hydrolases based on amino acid sequence similarities. Biochem. J. 280: 309-316.
Henrissat B. and Bairoch A. (1993) New families in the classification of glycosyl hydrolases based on amino acid sequence similarities. Biochem. J. 293: 781-788.
Henrissat B. and Bairoch A. (1996).Updating the sequence-based classification of glycosyl hydrolases. Biochem. J. 316: 695-696
IUPAC (International Union of Pure and Applied Chemistry) (1987). Measurement of cellulase activities. Pure and Appl. Chem. 59:257-268.
Joutsjoki, VV, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Lowry OH, NJ Roseborough, AL Farr and RJ Randall. 1951. Protein measurement with the Folin phenol reagent. J. Biol Chem 193: 265-275.
Malardier L, Daboussi MJ , Julien J, Roussel F, Scazzocchio C and Brygoo Y. 1989. Cloning of the nitrate reductase gene (niaD) of Aspergillus nidulans and its use for transformation of Fusarium oxysporum. Gene 15:147-156.
Needleman S. and Wunsch C. (1970) A general method applicable to the search for similarities in the amino acid sequence of two proteins. Journal of Molecular Biology 48, 443-453.
Nielsen H., J. Engelbrecht, S. Brunak, and G. von Heijne. 1997. Identification of prokaryotic and eukaryotic signal peptides and prediction of their cleavage sites. Prot. Engineering 10:1-6.
Nierstrasz V.A. and Warmoeskerken M.M.C.G. (2003) Process engineering and industrial enzyme applications. In: Textile processing with enzymes. A.Cavaco-Paulo and G.M.Gübitz (eds.) Woodhead Publishing Ltd, Cambridge. pp.120-157.
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Raeder U and P Broda. 1985. Rapid preparation of DNA from filamentous fungi. Lett. Appl. Microbiol. 1:17-20.
Rice P, Longden I and Bleasby A. (2000). EMBOSS: The European Molecular Biology Open Software Suite. Trends in Genetics 16:276-277.
Sambrook J, EF Fritsch and T Maniatis. 1989. Molecular cloning, a laboratory manual. Cold Spring Harbor Laboratory, New York, US.
Sambrook J and DW Russell. 2001. Molecular cloning, a laboratory manual. Cold Spring Harbor Laboratory, New York, US.
Ward M, Shan W, Dauberman J, Weiss G, Larenas E, Bower B, Rey M, Clarkson K and Bott R. (1993) Cloning, sequence and preliminary structural analysis of a small, high pi endoglucanase (EGIII) from Trichoderma reesei. Proceedings of the second TRICEL symposium on TRICHODERMA REESEI CELLULASES AND OTHER HYDROLASES, Espoo, Finland, 1993, ed. by P. Suominen and T. Reinikainen. Foundation for Biotechnical and Industrial Fermentation Research 8 (1993): 153-158.

## Claims

1. An endoglucanase polypeptide comprising cellulolytic activity and being selected from the group consisting of:
a) a polypeptide comprising an amino acid sequence having at least 95% identity to SEQ ID NO: 2, and
b) a polypeptide of a) lacking the carbohydrate binding domain, or the carbohydrate binding domain and the linker region.

2. The endoglucanase polypeptide of claim 1, wherein said amino acid sequence has at least 98% sequence identity to SEQ ID NO: 2.

3. The endoglucanase polypeptide of claim 1, which has the amino acid sequence of SEQ ID NO: 17 or SEQ ID NO: 19.

4. The endoglucanase polypeptide of claim 1, which is obtainable or originates from the *Acremonium sp.,* preferably from *Acremonium thermophilum,* most preferably from *Acremonium sp.* CBS 116240.

5. An endoglucanase fusion protein comprising the cellulolytically active core of a polypeptide having at least 95% sequence identity to SEQ ID NO 2, attached to a heterologous carbohydrate binding domain (CBD).

6. The endoglucanase fusion protein of claim 5, wherein said CBD and an optional linker region are derived from *Trichoderma reesei* CBHI, *Chaetomium thermophilum* CBHI, or *Acremonium sp.* xylanase.

7. The endoglucanase fusion protein of claim 6, wherein the fusion protein has an amino acid sequence selected from the group consisting of an amino acid sequence having at least 71% sequence identity to SEQ ID NO: 39, at least 69% sequence identity to SEQ ID NO: 40, and at least 69% sequence identity to SEQ ID NO: 41.

8. An isolated polynucleotide encoding the endoglucanase polypeptide of any one of claims 1 to 4 or an endoglucanase fusion protein of any one of claims 5 to 7.

9. The isolated polynucleotide of claim 8 having a nucleotide sequence selected from the group consisting of:
a) a nucleotide sequence of SEQ ID NO: 1, SEQ ID NO: 16, SEQ ID NO: 18, SEQ ID NO: 36, SEQ ID NO: 37, or SEQ ID NO: 38;
b) a complementary strand of a); and
c) a sequence that is degenerate as a result of the genetic code to any one of the sequences as defined in a) or b).

10. An expression vector comprising a polynucleotide sequence encoding the endoglucanase polypeptide of any one of claims 1 to 4 or an endoglucanase fusion protein of any one of claims 5 to 7.

11. A host cell comprising an expression vector of claim 10.

12. The host cell of claim 11, which is of fungal origin.

13. The host cell of claim 12, which is *Trichoderma reesei.*

14. A process for the production of an endoglucanase polypeptide of any one of claims 1 to 4 or an endoglucanase fusion protein of any one of claims 5 to 7 comprising the step of culturing the host cell of any one of claims 11 to 13.

15. An enzyme preparation comprising an endoglucanase polypeptide of any one of claims 1 to 4 or an endoglucanase fusion protein of any one of claims 5 to 7.

16. A process for biostoning which comprises the step of adding an endoglucanase polypeptide of any one of claims 1 to 4 or an endoglucanase fusion protein of any one of claims 5 to 7 or a preparation of claim 15 to cotton containing fabric or garments, such as denim.

17. A process for biofinishing, which comprises the step of adding an endoglucanase polypeptide of any one of claims 1 to 4 or an endoglucanase fusion protein of any one of claims 5 to 7 or a preparation of claim 15 to textile materials like fabrics or garments or yarn.

18. A detergent composition comprising an endoglucanase polypeptide of any one of claims 1 to 4 or an endoglucanase fusion protein of any one of claims 5 to 7 or a preparation of claim 15 and auxiliaries, such as surface active agents, surfactants, bleaching agents or builders.

19. A method of treating cellulosic fiber containing textile material, wherein said method comprises contacting said textile material with the detergent composition of claim 18.

20. A method for treating wood-derived pulp or fiber, which comprises the step of adding an endoglucanase polypeptide of any one of claims 1 to 4 or an endoglucanase fusion protein of any one of claims 5 to 7 or a preparation of claim 15 to wood-derived mechanical or chemical pulp or secondary fiber.

21. A method for improving the quality of animal feed, which comprises treating plant material with an endoglucanase polypeptide of any one of claims 1 to 4 or an endoglucanase fusion protein of any one of claims 5 to 7 or a preparation of claim 15.

22. An *Escherichia coli* strain having accession number DSM 17324, DSM 18813, DSM 18814, DSM 18815, DSM 18816 or DSM 18817.

## Patentansprüche

1. Endoglucanasepolypeptid umfassend eine cellulolytische Aktivität und ausgewählt aus der Gruppe bestehend aus:
a) einem Polypeptid umfassend eine Aminosäuresequenz mit wenigstens 95 % Identität zu SEQ ID NO: 2, und
b) einem Polypeptid von a), welchem die Kohlenhydratbindungsdomäne oder die Kohlenhydratbindungsdomäne und die Linkerregion fehlt.

2. Endoglucanasepolypeptid nach Anspruch 1, wobei die Aminosäuresequenz wenigstens 98 % Sequenzidentität zu SEQ ID NO: 2 aufweist.

3. Endoglucanasepolypeptid nach Anspruch 1, welches die Aminosäuresequenz SEQ ID NO: 17 oder SEQ ID NO: 19 aufweist.

4. Endoglucanasepolypeptid nach Anspruch 1, welches von *Acremonium* sp, bevorzugt von *Acremonium thermophilum,* besonders bevorzugt von *Acremonium* sp. CBS 116240, erhältlich ist oder davon abgeleitet ist.

5. Endoglucanasefusionsprotein umfassend den cellulolytisch aktiven Kern eines Polypeptids mit wenigstens 95 % Sequenzidentität zu SEQ ID NO: 2, welches an eine heterologe Kohlenhydratbindungsdomäne (CBD) gebunden ist.

6. Endoglucanasefusionsprotein nach Anspruch 5, wobei die CBD und eine optionale Linkerregion von Xylanase von *Trichoderma reesei* CBHI, *Chaetomium thermophilum* CBHI oder *Acremonium* sp. abgeleitet sind.

7. Endoglucanasefusionsprotein nach Anspruch 6, wobei das Fusionsprotein eine Aminosäuresequenz aufweist, die ausgewählt ist aus der Gruppe bestehend aus einer Aminosäuresequenz, die wenigstens 71 % Sequenzidentität zu SEQ ID NO: 39, wenigstens 69 % Sequenzidentität zu SEQ ID NO: 40 und wenigstens 69 % Sequenzidentität zu SEQ ID NO: 41 aufweist.

8. Isoliertes Polynukleotid, welches das Endoglucanasepolypeptid nach einem der Ansprüche 1 bis 4 oder ein Endoglucanasefusionsprotein nach einem der Ansprüche 5 bis 7 kodiert.

9. Isoliertes Polynukleotid nach Anspruch 8, welches eine Nukleotidsequenz aufweist, welche ausgewählt ist aus der Gruppe bestehend aus:
a) einer Nukleotidsequenz der SEQ ID NO: 1, SEQ ID NO: 16, SEQ ID NO: 18, SEQ ID NO: 36, SEQ ID NO: 37 oder SEQ ID NO: 38;
b) einem komplementären Strang von a); und
c) einer Sequenz, die aufgrund des genetischen Codes zu einer der in a) oder b) definierten Sequenzen degeneriert ist.

10. Expressionsvektor umfassend eine Polynukleotidsequenz, die die Polynukleotidsequenz nach einem der Ansprüche 1 bis 4 oder ein Endoglucanasefusionsprotein nach einem der Ansprüche 5 bis 7 kodiert.

11. Wirtszelle umfassend einen Expressionsvektor nach Anspruch 10.

12. Wirtszelle nach Anspruch 11, welche fungalen Ursprungs ist.

13. Wirtszelle nach Anspruch 12, welche *Trichoderma reesei* ist.

14. Verfahren zur Herstellung eines Endoglucanasepolypeptids nach einem der Ansprüche 1 bis 4 oder eines Endoglucanasefusionsproteins nach einem der Ansprüche 5 bis 7, umfassend den Schritt des Kultivierens der Wirtszellen nach einem der Ansprüche 11 bis 13.

15. Enzymzubereitung umfassend ein Endoglucanasepolypeptid nach einem der Ansprüche 1 bis 4 oder ein Endoglucanasefusionsprotein nach einem der Ansprüche 5 bis 7.

16. Verfahren zum Biostoning, welches den Schritt des Zugebens eines Endoglucanasepolypeptids nach einem der Ansprüche 1 bis 4 oder eines Endoglucanasefusionsproteins nach einem der Ansprüche 5 bis 7 oder einer Zubereitung nach Anspruch 15 zu baumwollhaltigen Stoffen oder Kleidungsstücken, z.B. Denim, umfasst.

17. Verfahren zum Biofinishing, welches den Schritt des Zugebens eines Endoglucanasepolypeptids nach einem der Ansprüche 1 bis 4 oder eines Endoglucanasefusionsproteins nach einem der Ansprüche 5 bis 7 oder einer Zubereitung nach Anspruch 15 zu Textilmaterialien wie Stoffen oder Kleidungsstücken oder Garn umfasst.

18. Detergenzzusammensetzung umfassend ein Endoglucanasepolypeptid nach einem der Ansprüche 1 bis 4 oder ein Endoglucanasefusionsprotein nach einem der Ansprüche 5 bis 7 oder eine Zubereitung nach Anspruch 15 und Hilfsstoffe wie z.B. oberflächenaktiven Stoffe, Tenside, Bleichmittel oder Builder.

19. Verfahren zum Behandeln von zellulosefaserhaltigem Textilmaterial, wobei das Verfahren ein Kontaktieren des Textilmaterials mit der Detergenzzusammensetzung nach Anspruch 18 umfasst.

20. Verfahren zum Behandeln von holzbasierter Pulpe oder Faser, welches den Schritt des Zugebens eines Endoglucanasepolypeptids nach einem der Ansprüche 1 bis 4 oder eines Endoglucanasefusionsproteins nach einem der Ansprüche 5 bis 7 oder einer Zubereitung nach Anspruch 15 zu holzbasierter mechanischer oder chemischer Pulpe oder sekundärer Faser umfasst.

21. Verfahren zum Verbessern der Qualität von Tiernahrung, welches ein Behandeln von Pflanzenmaterial mit einem Endoglucanasepolypeptid nach einem der Ansprüche 1 bis 4 oder einem Endoglucanasefusionsprotein nach einem der Ansprüche 5 bis 7 oder einer Zubereitung nach Anspruch 15 umfasst.

22. *Escherichia coli* Stamm, welcher die Zugangsnummer DSM 17324, DSM 18813, DSM 18814, DSM 18815, DSM 18816 oder DSM 18817 aufweist.

## Revendications

1. Polypeptide d'endoglucanase comprenant une activité cellulolytique et étant choisi dans le groupe constitué par :
a) un polypeptide comprenant une séquence d'acides aminés ayant au moins 95% d'identité avec SEQ ID N°:2, et
b) un polypeptide de a) dépourvu du domaine de liaison aux glucides, ou du domaine de liaison aux glucides et de région de liaison.

2. Polypeptide d'endoglucanase selon la revendication 1, dans lequel ladite séquence d'acides aminés a au moins 98% d'identité de séquence avec SEQ ID N°:2.

3. Polypeptide d'endoglucanase selon la revendication 1, qui a la séquence d'acides aminés SEQ ID N°:17 ou SEQ ID N°:19.

4. Polypeptide d'endoglucanase selon la revendication 1, pouvant être obtenu à partir de ou qui provient d'*Acremonium sp.,* de préférence d'*Acremonium thermophilum,* plus préférentiellement d'*Acremonium sp.* CBS 116240.

5. Protéine de fusion d'endoglucanase comprenant l'activité principale cellulolytique d'un polypeptide ayant 95% d'identité de séquence avec SEQ ID N°:2, attaché à un domaine de liaison aux glucides hétérologue (CDB).

6. Protéine de fusion d'endoglucanase selon la revendication 5, dans laquelle ledit CDB et une région de liaison optionnelle sont dérivés à partir de *Trichoderma reesiei* CBHI, de *Chaetomium thermophilum* CBHI, ou d'*Adremonium sp.* xylanase.

7. Protéine de fusion d'endoglucanase selon la revendication 6, dans laquelle la protéine de fusion a une séquence d'acides aminés choisie dans le groupe constitué par une séquence d'acides aminés ayant au moins 71% d'identité de séquence avec SEQ ID N°:39, au moins 69% d'identité de séquence avec SEQ ID N°:40, et au moins 69% d'identité de séquence avec SEQ ID N°:41.

8. Polynucléotide isolé codant pour le polypeptide d'endoglucanase selon l'une quelconque des revendications 1 à 4 ou pour une protéine de fusion d'endoglucanase selon l'une quelconque des revendications 5 à 7.

9. Polynucléotide isolé selon la revendication 8 ayant une séquence nucléotidique choisie dans le groupe constitué par :
a) une séquence nucléotidique SED ID N°:1, SEQ ID N°:16, SEQ ID N°:18, SEQ ID N°:36, SEQ ID N°:37, ou SEQ ID N°:38 ;
b) un brin complémentaire de a) ; et
c) une séquence qui est dégénérée en résultat du code génétique en l'une quelconque des séquences telles que définies dans a) ou b).

10. Vecteur d'expression comprenant un polypeptide d'endoglucanase selon l'une quelconque des revendications 1 à 4 ou une protéine de fusion d'endoglucanase selon l'une quelconque des revendications 5 à 7.

11. Cellule hôte comprenant un vecteur d'expression selon la revendication 10.

12. Cellule hôte selon la revendication 12, laquelle est d'origine fongique.

13. Cellule hôte selon la revendication 12, laquelle est *Trichoderma reesiei.*

14. Procédé de production d'un polypeptide d'endoglucanase selon l'une quelconque des revendications 1 à 4 ou d'une protéine de fusion d'endoglucanase selon l'une quelconque des revendications 5 à 7 comprenant l'étape de culture de la cellule hôte selon l'une quelconque des revendications 11 à 13.

15. Préparation enzymatique comprenant un polypeptide d'endoglucanase selon l'une quelconque des revendications 1 à 4 ou une protéine de fusion d'endoglucanase selon l'une quelconque des revendications 5 à 7.

16. Procédé de biodélavage comprenant l'étape d'ajout d'un polypeptide d'endoglucanase selon l'une quelconque des revendications 1 à 4 ou d'une protéine de fusion d'endoglucanase selon l'une quelconque des revendications 5 à 7 ou d'une préparation selon la revendication 15 à un tissu ou des vêtements comprenant du coton, tel que du denim.

17. Procédé de traitement biologique de finition comprenant l'étape d'ajout d'un polypeptide d'endoglucanase selon l'une quelconque des revendications 1 à 4 ou d'une protéine de fusion d'endoglucanase selon l'une quelconque des revendications 5 à 7 ou d'une préparation selon la revendication 15 à des matières textiles, telles que des tissus, des vêtements ou du fil.

18. Composition détergente comprenant un polypeptide d'endoglucanase selon l'une quelconque des revendications 1 à 4 ou une protéine de fusion d'endoglucanase selon l'une quelconque des revendications 5 à 7 ou une préparation selon la revendication 15 et des additifs, tels que des agents tensioactifs, des tensioactifs, des agents blanchissants ou des adjuvants.

19. Procédé de traitement d'une matière textile contenant des fibres cellulosiques, dans lequel ledit procédé comprend une mise en contact de ladite matière textile avec la composition détergente selon la revendication 18.

20. Procédé de traitement d'une pâte à papier ou de fibres dérivées du bois, comprenant l'étape d'ajout d'un polypeptide d'endoglucanase selon l'une quelconque des revendications 1 à 4 ou d'une protéine de fusion d'endoglucanase selon l'une quelconque des revendications 5 à 7 ou d'une préparation selon la revendication 15 à une pâte à papier chimique ou mécanique dérivée du bois ou à des fibres secondaires.

21. Procédé pour améliorer la qualité de la nourriture pour animaux, comprenant un traitement d'une matière végétale par un polypeptide d'endoglucanase selon l'une quelconque des revendications 1 à 4 ou une protéine de fusion d'endoglucanase selon l'une quelconque des revendications 5 à 7 ou une préparation selon la revendication 15.

22. Souche d'*Escherichia coli* ayant un numéro d'enregistrement DSM 17324, DSM 18813, DSM 18814, DSM 18815, DSM 18816 ou DSM 18817.
